# EUROPÄISCHE PATENTSCHRIFT

(11) **EP 3 625 336 B1**
(45) Veröffentlichungstag und Bekanntmachung des Hinweises auf die Patenterteilung: **07.07.2021**
(21) Anmeldenummer: 18721939.9
(22) Anmeldetag: 13.04.2018
(51) Int. Cl.: C12N 9/00, C12N 9/12, C12N 15/52, C12N 15/63, C12N 15/74

(54) **PYRUVATCARBOXYLASE UND FÜR DIE PYRUVATCARBOXYLASE KODIERENDE DNA, PLASMID ENTHALTEND DIE DNA, SOWIE MIKROORGANISMUS ZUR PRODUKTION UND VERFAHREN ZUR HERSTELLUNG VON PRODUKTEN, DEREN BIOSYNTHESE OXALACETAT ALS VORSTUFE BEINHALTET UND CHROMOSOM**
PYRUVATE CARBOXYLASE AND PYRUVATE CARBOXYLASE-ENCODING DNA, PLASMID CONTAINING SAID DNA AND MICROORGANISM FOR THE PRODUCTION THEREOF, AND METHODS FOR THE PRODUCTION OF PRODUCTS THE BIOSYNTHESIS OF WHICH INCLUDES OXALOACETATE AS PRECURSOR, AND CHROMOSOME
PYRUVATE CARBOXYLASE ET ADN CODANT POUR LA PYRUVATE CARBOXYLASE, PLASMIDE CONTENANT LEDIT ADN, ET MICRO-ORGANISME DE PRODUCTION ET PROCÉDÉ POUR LA PRÉPARATION DE PRODUITS DONT LA BIOSYNTHÈSE COMPREND L'OXALACÉTATE COMME PRÉCURSEUR, ET CHROMOSOME

(30) Priorität: 18.05.2017 DE 102017004751
(43) Veröffentlichungstag der Anmeldung: 25.03.2020
(73) Patentinhaber: Forschungszentrum Jülich GmbH, 52425 Jülich (DE)
(72) Erfinder: KORTMANN, Maike, 52066 Aachen (DE); BAUMGART, Meike, 50129 Bergheim (DE); BOTT, Michael, 52428 J lich (DE)
(86) Internationale Anmeldenummer: PCT/DE2018/000104
(87) Internationale Veröffentlichungsnummer: WO 2018/210358

(56) Entgegenhaltungen:
- EP-A1- 1 067 193
- WO-A2-2008/006680
- DE-A1- 19 831 609

### SEQUENCE LISTING

<110> Forschungszentrum Jülich GmbH
<120> Pyruvatcarboxylase und für die Pyruvatcarboxylase kodierende DNA, Plasmid enthaltend die DNA, sowie Mikroorganismus zur Produktion und Verfahren zur Herstellung von Produkten, deren Biosynthese Oxalacetat als Vorstufe beinhaltet und Chromosom
<130> PT1.2785
<160> 8
<170> PatentIn version 3.5
<210> 1
   <211> 3423
   <212> DNA
   <213> Corynebacterium glutamicum
<400> 1
   atgtcgactc acacatcttc aacgcttcca gcattcaaaa agatcttggt agcaaaccgc      60
   ggcgaaatcg cggtccgtgc tttccgtgca gcactcgaaa ccggtgcagc cacggtagct     120
   atttaccccc gtgaagatcg gggatcattc caccgctctt ttgcttctga agctgtccgc     180
   attggtaccg aaggctcacc agtcaaggcg tacctggaca tcgatgaaat tatcggtgca     240
   gctaaaaaag ttaaagcaga tgccatttac ccgggatacg gcttcctgtc tgaaaatgcc     300
   cagcttgccc gcgagtgtgc ggaaaacggc attactttta ttggcccaac cccagaggtt     360
   cttgatctca ccggtgataa gtctcgcgcg gtaaccgccg cgaagaaggc tggtctgcca     420
   gttttggcgg aatccacccc gagcaaaaac atcgatgaga tcgttaaaag cgctgaaggc     480
   cagacttacc ccatctttgt gaaggcagtt gccggtggtg gcggacgcgg tatgcgtttt     540
   gttgcttcac ctgatgagct tcgcaaatta gcaacagaag catctcgtga agctgaagcg     600
   gctttcggcg atggcgcggt atatgtcgaa cgtgctgtga ttaaccctca gcatattgaa     660
   gtgcagatcc ttggcgatca cactggagaa gttgtacacc tttatgaacg tgactgctca     720
   ctgcagcgtc gtcaccaaaa agttgtcgaa attgcgccag cacagcattt ggatccagaa     780
   ctgcgtgatc gcatttgtgc ggatgcagta aagttctgcc gctccattgg ttaccagggc     840
   gcgggaaccg tggaattctt ggtcgatgaa aagggcaacc acgtcttcat cgaaatgaac     900
   ccacgtatcc aggttgagca caccgtgact gaagaagtca ccgaggtgga cctggtgaag     960
   gcgcagatgc gcttggctgc tggtgcaacc ttgaaggaat tgggtctgac ccaagataag    1020
   atcaaggccc acggtgcagc actgcagtgc cgcatcacca cggaagatcc aaacaacggc    1080
   ttccgcccag ataccggaac tatcaccgcg taccgctcac caggcggagc tggcgttcgt    1140
   cttgacggtg cagctcagct cggtggcgaa atcaccgcac actttgactc catgctggtg    1200
   aaaatgacct gccgtggttc cgactttgaa actgctgttg ctcgtgcaca gcgcgcgttg    1260
   gctgagttca ccgtgtctgg tgttgcaacc aacattggtt tcttgcgtgc gttgctgcgg 1320
   gaagaggact tcacttccaa gcgcatcgcc accggattca ttgccgatca cccgcacctc 1380
   cttcaggctc cacctgctga tgatgagcag ggacgcatcc tggattactt ggcagatgtc 1440
   accgtgaaca agcctcatgg tgtgcgtcca aaggatgttg cagctcctat cgataagctg 1500
   cctaacatca aggatctgcc actgccacgc ggttcccgtg accgcctgaa gcagcttggc 1560
   ccagccgcgt ttgctcgtga tctccgtgag caggacgcac tggcagttac tgataccacc 1620
   ttccgcgatg cacaccagtc tttgcttgcg acccgagtcc gctcattcgc actgaagcct 1680
   gcggcagagg ccgtcgcaaa gctgactcct gagcttttgt ccgtggaggc ctggggcggc 1740
   gcgacctacg atgtggcgat gcgtttcctc tttgaggatc cgtgggacag gctcgacgag 1800
   ctgcgcgagg cgatgccgaa tgtaaacatt cagatgctgc ttcgcggccg caacaccgtg 1860
   ggatacaccc cgtacccaga ctccgtctgc cgcgcgtttg ttaaggaagc tgccagctcc 1920
   ggcgtggaca tcttccgcat cttcgacgcg cttaacgacg tctcccagat gcgtccagca 1980
   atcgacgcag tcctggagac caacaccgcg gtagccgagg tggctatggc ttattctggt 2040
   gatctctctg atccaaatga aaagctctac accctggatt actacctaaa gatggcagag 2100
   gagatcgtca agtctggcgc tcacatcttg gccattaagg atatggctgg tctgcttcgc 2160
   ccagctgcgg taaccaagct ggtcaccgca ctgcgccgtg aattcgatct gccagtgcac 2220
   gtgcacaccc acgacactgc gggtggccag ctggcaacct actttgctgc agctcaagct 2280
   ggtgcagatg ctgttgacgg tgcttccgca ccactgtctg gcaccacctc ccagccatcc 2340
   ctgtctgcca ttgttgctgc attcgcgcac acccgtcgcg ataccggttt gagcctcgag 2400
   gctgtttctg acctcgagcc gtactgggaa gcagtgcgcg gactgtacct gccatttgag 2460
   tctggaaccc caggcccaac cggtcgcgtc taccgccacg aaatcccagg cggacagttg 2520
   tccaacctgc gtgcacaggc caccgcactg ggccttgcgg atcgtttcga actcatcgaa 2580
   gacaactacg cagccgttaa tgagatgctg ggacgcccaa ccaaggtcac cccatcctcc 2640
   aaggttgttg gcgacctcgc actccacctc gttggtgcgg gtgtggatcc agcagacttt 2700
   gctgccgatc cacaaaagta cgacatccca gactctgtca tcgcgttcct gcgcggcgag 2760
   cttggtaacc ctccaggtgg ctggccagag ccactgcgca cccgcgcact ggaaggccgc 2820
   tccgaaggca aggcacctct gacggaagtt cctgaggaag agcaggcgca cctcgacgct 2880
   gatgattcca aggaacgtcg caatagcctc aaccgcctgc tgttcccgaa gccaaccgaa 2940
   gagttcctcg agcaccgtcg ccgcttcggc aacacctctg cgctggatga tcgtgaattc 3000
   ttctacggcc tggtcgaagg ccgcgagact ttgatccgcc tgccagatgt gcgcacccca 3060
   ctgcttgttc gcctggatgc gatctctgag ccagacgata agggtatgcg caatgttgtg 3120
   gccaacgtca acggccagat ccgcccaatg cgtgtgcgtg accgctccgt tgagtctgtc 3180
   accgcaaccg cagaaaaggc agattcctcc aacaagggcc atgttgctgc accattcgct 3240
   ggtgttgtca ccgtgactgt tgctgaaggt gatgaggtca aggctggaga tgcagtcgca 3300
   atcatcgagg ctatgaagat ggaagcaaca atcactgctt ctgttgacgg caaaatcgat 3360
   cgcgttgtgg ttcctgctgc aacgaaggtg gaaggtggcg acttgatcgt cgtcgtttcc 3420
   taa 3423
<210> 2
   <211> 3423
   <212> DNA
   <213> Corynebacterium glutamicum
<400> 2
   atgtcgactc acacatcttc aacgcttcca gcattcaaaa agatcttggt agcaaaccgc      60
   ggcgaaatcg cggtccgtgc tttccgtgca gcactcgaaa ccggtgcagc cacggtagct     120
   atttaccccc gtgaagatcg gggatcattc caccgctctt ttgcttctga agctgtccgc     180
   attggtaccg aaggctcacc agtcaaggcg tacctggaca tcgatgaaat tatcggtgca     240
   gctaaaaaag ttaaagcaga tgccatttac ccgggatacg gcttcctgtc tgaaaatgcc     300
   cagcttgccc gcgagtgtgc ggaaaacggc attactttta ttggcccaac cccagaggtt     360
   cttgatctca ccggtgataa gtctcgcgcg gtaaccgccg cgaagaaggc tggtctgcca     420
   gttttggcgg aatccacccc gagcaaaaac atcgatgaga tcgttaaaag cgctgaaggc     480
   cagacttacc ccatctttgt gaaggcagtt gccggtggtg gcggacgcgg tatgcgtttt     540
   gttgcttcac ctgatgagct tcgcaaatta gcaacagaag catctcgtga agctgaagcg     600
   gctttcggcg atggcgcggt atatgtcgaa cgtgctgtga ttaaccctca gcatattgaa     660
   gtgcagatcc ttggcgatca cactggagaa gttgtacacc tttatgaacg tgactgctca     720
   ctgcagcgtc gtcaccaaaa agttgtcgaa attgcgccag cacagcattt ggatccagaa     780
   ctgcgtgatc gcatttgtgc ggatgcagta aagttctgcc gctccattgg ttaccagggc     840
   gcgggaaccg tggaattctt ggtcgatgaa aagggcaacc acgtcttcat cgaaatgaac     900
   ccacgtatcc aggttgagca caccgtgact gaagaagtca ccgaggtgga cctggtgaag     960
   gcgcagatgc gcttggctgc tggtgcaacc ttgaaggaat tgggtctgac ccaagataag    1020
   atcaaggccc acggtgcagc actgcagtgc cgcatcacca cggaagatcc aaacaacggc    1080
   ttccgcccag ataccggaac tatcaccgcg taccgctcac caggcggagc tggcgttcgt    1140
   cttgacggtg cagctcagct cggtggcgaa atcaccgcac actttgactc catgctggtg    1200
   aaaatgacct gccgtggttc cgactttgaa actgctgttg ctcgtgcaca gcgcgcgttg    1260
   gctgagttca ccgtgtctgg tgttgcaacc aacattggtt tcttgcgtgc gttgctgcgg    1320
   gaagaggact tcacttccaa gcgcatcgcc accggattca ttgccgatca cccgcacctc    1380
   cttcaggctc cacctgctga tgatgagcag ggacgcatcc tggattactt ggcagatgtc 1440
   accgtgaaca agcctcatgg tgtgcgtcca aaggatgttg cagctcctat cgataagctg 1500
   cctaacatca aggatctgcc actgccacgc ggttcccgtg accgcctgaa gcagcttggc 1560
   ccagccgcgt ttgctcgtga tctccgtgag caggacgcac tggcagttac tgataccacc 1620
   ttccgcgatg cacaccagtc tttgcttgcg acccgagtcc gctcattcgc actgaagcct 1680
   gcggcagagg ccgtcgcaaa gctgactcct gagcttttgt ccgtggaggc ctggggcggc 1740
   gcgacctacg atgtggcgat gcgtttcctc tttgaggatc cgtgggacag gctcgacgag 1800
   ctgcgcgagg cgatgccgaa tgtaaacatt cagatgctgc ttcgcggccg caacaccgtg 1860
   ggatacaccc cgtacccaga ctccgtctgc cgcgcgtttg ttaaggaagc tgccagctcc 1920
   ggcgtggaca tcttccgcat cttcgacgcg cttaacgacg tctcccagat gcgtccagca 1980
   atcgacgcag tcctggagac caacaccgcg gtagccgagg tggctatggc ttattctggt 2040
   gatctctctg atccaaatga aaagctctac accctggatt actacctaaa gatggcagag 2100
   gagatcgtca agtctggcgc tcacatcttg gccattaagg atatggctgg tctgcttcgc 2160
   ccagctgcgg taaccaagct ggtcaccgca ctgcgccgtg aattcgatct gccagtgcac 2220
   gtgcacaccc acgacactgc gggtggccag ctggcaacct actttgctgc agctcaagct 2280
   ggtgcagatg ctgttgacgg tgcttccgca ccactgtctg gcaccacctc ccagccatcc 2340
   ctgtctgcca ttgttgctgc attcgcgcac acccgtcgcg ataccggttt gagcctcgag 2400
   gctgtttctg acctcgagcc gtactgggaa gcagtgcgcg gactgtacct gccatttgag 2460
   tctggaaccc caggcccaac cggtcgcgtc taccgccacg aaatcccagg cggacagttg 2520
   tccaacctgc gtgcacaggc caccgcactg ggccttgcgg atcgtttcga actcatcgaa 2580
   gacaactacg cagccgttaa tgagatgctg ggacgcccaa ccaaggtcac cccatcctcc 2640
   aaggttgttg gcgacctcgc actccacctc gttggtgcgg gtgtggatcc agcagacttt 2700
   gctgccgatc cacaaaagta cgacatccca gactctgtca tcgcgttcct gcgcggcgag 2760
   cttggtaacc ctccaggtgg ctggccagag ccactgcgca cccgcgcact ggaaggccgc 2820
   tccgaaggca aggcacctct gacggaagtt cctgaggaag agcaggcgca cctcgacgct 2880
   gatgattcca aggaacgtcg caatagcctc aaccgcctgc tgttcccgaa gccaaccgaa 2940
   gagttcctcg agcaccgtcg ccgcttcggc aacacctctg cgctggatga tcgtgaattc      3000
   ttctacggcc tggtcgaagg ccgcgagact ttgagccgcc tgccagatgt gcgcacccca      3060
   ctgcttgttc gcctggatgc gatctctgag ccagacgata agggtatgcg caatgttgtg 3120
   gccaacgtca acggccagat ccgcccaatg cgtgtgcgtg accgctccgt tgagtctgtc 3180
   accgcaaccg cagaaaaggc agattcctcc aacaagggcc atgttgctgc accattcgct 3240
   ggtgttgtca ccgtgactgt tgctgaaggt gatgaggtca aggctggaga tgcagtcgca 3300
   atcatcgagg ctatgaagat ggaagcaaca atcactgctt ctgttgacgg caaaatcgat 3360
   cgcgttgtgg ttcctgctgc aacgaaggtg gaaggtggcg acttgatcgt cgtcgtttcc 3420
   taa 3423
<210> 3
   <211> 1140
   <212> PRT
   <213> Corybebacterium glutamicum
<400> 3
   Met Ser Thr His Thr Ser Ser Thr Leu Pro Ala Phe Lys Lys Ile Leu
   1 5 10 15
   Val Ala Asn Arg Gly Glu Ile Ala Val Arg Ala Phe Arg Ala Ala Leu
   20 25 30
   Glu Thr Gly Ala Ala Thr Val Ala Ile Tyr Pro Arg Glu Asp Arg Gly
   35 40 45
   Ser Phe His Arg Ser Phe Ala Ser Glu Ala Val Arg Ile Gly Thr Glu
   50 55 60
   Gly Ser Pro Val Lys Ala Tyr Leu Asp Ile Asp Glu Ile Ile Gly Ala
   65 70 75 80
   Ala Lys Lys Val Lys Ala Asp Ala Ile Tyr Pro Gly Tyr Gly Phe Leu
   85 90 95
   Ser Glu Asn Ala Gln Leu Ala Arg Glu Cys Ala Glu Asn Gly Ile Thr
   100 105 110
   Phe Ile Gly Pro Thr Pro Glu Val Leu Asp Leu Thr Gly Asp Lys Ser
   115 120 125
   Arg Ala Val Thr Ala Ala Lys Lys Ala Gly Leu Pro Val Leu Ala Glu
   130 135 140
   Ser Thr Pro Ser Lys Asn Ile Asp Glu Ile Val Lys Ser Ala Glu Gly
   145 150 155 160
   Gln Thr Tyr Pro Ile Phe Val Lys Ala Val Ala Gly Gly Gly Gly Arg
   165 170 175
   Gly Met Arg Phe Val Ala Ser Pro Asp Glu Leu Arg Lys Leu Ala Thr
   180 185 190
   Glu Ala Ser Arg Glu Ala Glu Ala Ala Phe Gly Asp Gly Ala Val Tyr
           195                 200                 205
   Val Glu Arg Ala Val Ile Asn Pro Gln His Ile Glu Val Gln Ile Leu
       210                 215                 220
   Gly Asp His Thr Gly Glu Val Val His Leu Tyr Glu Arg Asp Cys Ser
   225                 230                 235                 240
   Leu Gln Arg Arg His Gln Lys Val Val Glu Ile Ala Pro Ala Gln His
                   245                 250                 255
   Leu Asp Pro Glu Leu Arg Asp Arg Ile Cys Ala Asp Ala Val Lys Phe
               260                 265                 270
   Cys Arg Ser Ile Gly Tyr Gln Gly Ala Gly Thr Val Glu Phe Leu Val
           275                 280                 285
   Asp Glu Lys Gly Asn His Val Phe Ile Glu Met Asn Pro Arg Ile Gln
       290                 295                 300
   Val Glu His Thr Val Thr Glu Glu Val Thr Glu Val Asp Leu Val Lys
   305                 310                 315                 320
   Ala Gln Met Arg Leu Ala Ala Gly Ala Thr Leu Lys Glu Leu Gly Leu
                   325                 330                 335
   Thr Gln Asp Lys Ile Lys Ala His Gly Ala Ala Leu Gln Cys Arg Ile
               340                 345                 350
   Thr Thr Glu Asp Pro Asn Asn Gly Phe Arg Pro Asp Thr Gly Thr Ile
           355                 360                 365
   Thr Ala Tyr Arg Ser Pro Gly Gly Ala Gly Val Arg Leu Asp Gly Ala
       370                 375                 380
   Ala Gln Leu Gly Gly Glu Ile Thr Ala His Phe Asp Ser Met Leu Val
   385                 390                 395                 400
   Lys Met Thr Cys Arg Gly Ser Asp Phe Glu Thr Ala Val Ala Arg Ala
                   405                 410                 415
   Gln Arg Ala Leu Ala Glu Phe Thr Val Ser Gly Val Ala Thr Asn Ile
               420                 425                 430
   Gly Phe Leu Arg Ala Leu Leu Arg Glu Glu Asp Phe Thr Ser Lys Arg
           435                 440                 445
   Ile Ala Thr Gly Phe Ile Ala Asp His Pro His Leu Leu Gln Ala Pro
   450 455 460
   Pro Ala Asp Asp Glu Gln Gly Arg Ile Leu Asp Tyr Leu Ala Asp Val
   465 470 475 480
   Thr Val Asn Lys Pro His Gly Val Arg Pro Lys Asp Val Ala Ala Pro
   485 490 495
   Ile Asp Lys Leu Pro Asn Ile Lys Asp Leu Pro Leu Pro Arg Gly Ser
   500 505 510
   Arg Asp Arg Leu Lys Gln Leu Gly Pro Ala Ala Phe Ala Arg Asp Leu
   515 520 525
   Arg Glu Gln Asp Ala Leu Ala Val Thr Asp Thr Thr Phe Arg Asp Ala
   530 535 540
   His Gln Ser Leu Leu Ala Thr Arg Val Arg Ser Phe Ala Leu Lys Pro
   545 550 555 560
   Ala Ala Glu Ala Val Ala Lys Leu Thr Pro Glu Leu Leu Ser Val Glu
   565 570 575
   Ala Trp Gly Gly Ala Thr Tyr Asp Val Ala Met Arg Phe Leu Phe Glu
   580 585 590
   Asp Pro Trp Asp Arg Leu Asp Glu Leu Arg Glu Ala Met Pro Asn Val
   595 600 605
   Asn Ile Gln Met Leu Leu Arg Gly Arg Asn Thr Val Gly Tyr Thr Pro
   610 615 620
   Tyr Pro Asp Ser Val Cys Arg Ala Phe Val Lys Glu Ala Ala Ser Ser
   625 630 635 640
   Gly Val Asp Ile Phe Arg Ile Phe Asp Ala Leu Asn Asp Val Ser Gln
   645 650 655
   Met Arg Pro Ala Ile Asp Ala Val Leu Glu Thr Asn Thr Ala Val Ala
   660 665 670
   Glu Val Ala Met Ala Tyr Ser Gly Asp Leu Ser Asp Pro Asn Glu Lys
   675 680 685
   Leu Tyr Thr Leu Asp Tyr Tyr Leu Lys Met Ala Glu Glu Ile Val Lys
       690                 695                 700
   Ser Gly Ala His Ile Leu Ala Ile Lys Asp Met Ala Gly Leu Leu Arg
   705                 710                 715                 720
   Pro Ala Ala Val Thr Lys Leu Val Thr Ala Leu Arg Arg Glu Phe Asp
                   725                 730                 735
   Leu Pro Val His Val His Thr His Asp Thr Ala Gly Gly Gln Leu Ala
               740                 745                 750
   Thr Tyr Phe Ala Ala Ala Gln Ala Gly Ala Asp Ala Val Asp Gly Ala
           755                 760                 765
   Ser Ala Pro Leu Ser Gly Thr Thr Ser Gln Pro Ser Leu Ser Ala Ile
       770                 775                 780
   Val Ala Ala Phe Ala His Thr Arg Arg Asp Thr Gly Leu Ser Leu Glu
   785                 790                 795                 800
   Ala Val Ser Asp Leu Glu Pro Tyr Trp Glu Ala Val Arg Gly Leu Tyr
                   805                 810                 815
   Leu Pro Phe Glu Ser Gly Thr Pro Gly Pro Thr Gly Arg Val Tyr Arg
               820                 825                 830
   His Glu Ile Pro Gly Gly Gln Leu Ser Asn Leu Arg Ala Gln Ala Thr
           835                 840                 845
   Ala Leu Gly Leu Ala Asp Arg Phe Glu Leu Ile Glu Asp Asn Tyr Ala
       850                 855                 860
   Ala Val Asn Glu Met Leu Gly Arg Pro Thr Lys Val Thr Pro Ser Ser
   865                 870                 875                 880
   Lys Val Val Gly Asp Leu Ala Leu His Leu Val Gly Ala Gly Val Asp
                   885                 890                 895
   Pro Ala Asp Phe Ala Ala Asp Pro Gln Lys Tyr Asp Ile Pro Asp Ser
               900                 905                 910
   Val Ile Ala Phe Leu Arg Gly Glu Leu Gly Asn Pro Pro Gly Gly Trp
           915                 920                 925
   Pro Glu Pro Leu Arg Thr Arg Ala Leu Glu Gly Arg Ser Glu Gly Lys
       930                 935                 940
   Ala Pro Leu Thr Glu Val Pro Glu Glu Glu Gln Ala His Leu Asp Ala
   945 950 955 960
   Asp Asp Ser Lys Glu Arg Arg Asn Ser Leu Asn Arg Leu Leu Phe Pro
   965 970 975
   Lys Pro Thr Glu Glu Phe Leu Glu His Arg Arg Arg Phe Gly Asn Thr
   980 985 990
   Ser Ala Leu Asp Asp Arg Glu Phe Phe Tyr Gly Leu Val Glu Gly Arg
   995 1000 1005
   Glu Thr Leu Ile Arg Leu Pro Asp Val Arg Thr Pro Leu Leu Val
   1010 1015 1020
   Arg Leu Asp Ala Ile Ser Glu Pro Asp Asp Lys Gly Met Arg Asn
   1025 1030 1035
   Val Val Ala Asn Val Asn Gly Gln Ile Arg Pro Met Arg Val Arg
   1040 1045 1050
   Asp Arg Ser Val Glu Ser Val Thr Ala Thr Ala Glu Lys Ala Asp
   1055 1060 1065
   Ser Ser Asn Lys Gly His Val Ala Ala Pro Phe Ala Gly Val Val
   1070 1075 1080
   Thr Val Thr Val Ala Glu Gly Asp Glu Val Lys Ala Gly Asp Ala
   1085 1090 1095
   Val Ala Ile Ile Glu Ala Met Lys Met Glu Ala Thr Ile Thr Ala
   1100 1105 1110
   Ser Val Asp Gly Lys Ile Asp Arg Val Val Val Pro Ala Ala Thr
   1115 1120 1125
   Lys Val Glu Gly Gly Asp Leu Ile Val Val Val Ser
   1130 1135 1140
<210> 4
   <211> 1140
   <212> PRT
   <213> Corynebacterium glutamicum
<400> 4
   Met Ser Thr His Thr Ser Ser Thr Leu Pro Ala Phe Lys Lys Ile Leu
   1 5 10 15
   Val Ala Asn Arg Gly Glu Ile Ala Val Arg Ala Phe Arg Ala Ala Leu
               20                  25                  30
   Glu Thr Gly Ala Ala Thr Val Ala Ile Tyr Pro Arg Glu Asp Arg Gly
           35                  40                  45
   Ser Phe His Arg Ser Phe Ala Ser Glu Ala Val Arg Ile Gly Thr Glu
       50                  55                  60
   Gly Ser Pro Val Lys Ala Tyr Leu Asp Ile Asp Glu Ile Ile Gly Ala
   65                  70                  75                  80
   Ala Lys Lys Val Lys Ala Asp Ala Ile Tyr Pro Gly Tyr Gly Phe Leu
                   85                  90                  95
   Ser Glu Asn Ala Gln Leu Ala Arg Glu Cys Ala Glu Asn Gly Ile Thr
               100                 105                 110
   Phe Ile Gly Pro Thr Pro Glu Val Leu Asp Leu Thr Gly Asp Lys Ser
           115                 120                 125
   Arg Ala Val Thr Ala Ala Lys Lys Ala Gly Leu Pro Val Leu Ala Glu
       130                 135                 140
   Ser Thr Pro Ser Lys Asn Ile Asp Glu Ile Val Lys Ser Ala Glu Gly
   145                 150                 155                 160
   Gln Thr Tyr Pro Ile Phe Val Lys Ala Val Ala Gly Gly Gly Gly Arg
                   165                 170                 175
   Gly Met Arg Phe Val Ala Ser Pro Asp Glu Leu Arg Lys Leu Ala Thr
               180                 185                 190
   Glu Ala Ser Arg Glu Ala Glu Ala Ala Phe Gly Asp Gly Ala Val Tyr
           195                 200                 205
   Val Glu Arg Ala Val Ile Asn Pro Gln His Ile Glu Val Gln Ile Leu
       210                 215                 220
   Gly Asp His Thr Gly Glu Val Val His Leu Tyr Glu Arg Asp Cys Ser
   225                 230                 235                 240
   Leu Gln Arg Arg His Gln Lys Val Val Glu Ile Ala Pro Ala Gln His
                   245                 250                 255
   Leu Asp Pro Glu Leu Arg Asp Arg Ile Cys Ala Asp Ala Val Lys Phe
               260                 265                 270
   Cys Arg Ser Ile Gly Tyr Gln Gly Ala Gly Thr Val Glu Phe Leu Val
           275                 280                 285
   Asp Glu Lys Gly Asn His Val Phe Ile Glu Met Asn Pro Arg Ile Gln
       290                 295                 300
   Val Glu His Thr Val Thr Glu Glu Val Thr Glu Val Asp Leu Val Lys
   305                 310                 315                 320
   Ala Gln Met Arg Leu Ala Ala Gly Ala Thr Leu Lys Glu Leu Gly Leu
                   325                 330                 335
   Thr Gln Asp Lys Ile Lys Ala His Gly Ala Ala Leu Gln Cys Arg Ile
               340                 345                 350
   Thr Thr Glu Asp Pro Asn Asn Gly Phe Arg Pro Asp Thr Gly Thr Ile
           355                 360                 365
   Thr Ala Tyr Arg Ser Pro Gly Gly Ala Gly Val Arg Leu Asp Gly Ala
       370                 375                 380
   Ala Gln Leu Gly Gly Glu Ile Thr Ala His Phe Asp Ser Met Leu Val
   385                 390                 395                 400
   Lys Met Thr Cys Arg Gly Ser Asp Phe Glu Thr Ala Val Ala Arg Ala
                   405                 410                 415
   Gln Arg Ala Leu Ala Glu Phe Thr Val Ser Gly Val Ala Thr Asn Ile
               420                 425                 430
   Gly Phe Leu Arg Ala Leu Leu Arg Glu Glu Asp Phe Thr Ser Lys Arg
           435                 440                 445
   Ile Ala Thr Gly Phe Ile Ala Asp His Pro His Leu Leu Gln Ala Pro
       450                 455                 460
   Pro Ala Asp Asp Glu Gln Gly Arg Ile Leu Asp Tyr Leu Ala Asp Val
   465                 470                 475                 480
   Thr Val Asn Lys Pro His Gly Val Arg Pro Lys Asp Val Ala Ala Pro
                   485                 490                 495
   Ile Asp Lys Leu Pro Asn Ile Lys Asp Leu Pro Leu Pro Arg Gly Ser
               500                 505                 510
   Arg Asp Arg Leu Lys Gln Leu Gly Pro Ala Ala Phe Ala Arg Asp Leu
           515                 520                 525
   Arg Glu Gln Asp Ala Leu Ala Val Thr Asp Thr Thr Phe Arg Asp Ala
       530                 535                 540
   His Gln Ser Leu Leu Ala Thr Arg Val Arg Ser Phe Ala Leu Lys Pro
   545                 550                 555                 560
   Ala Ala Glu Ala Val Ala Lys Leu Thr Pro Glu Leu Leu Ser Val Glu
                   565                 570                 575
   Ala Trp Gly Gly Ala Thr Tyr Asp Val Ala Met Arg Phe Leu Phe Glu
               580                 585                 590
   Asp Pro Trp Asp Arg Leu Asp Glu Leu Arg Glu Ala Met Pro Asn Val
           595                 600                 605
   Asn Ile Gln Met Leu Leu Arg Gly Arg Asn Thr Val Gly Tyr Thr Pro
       610                 615                 620
   Tyr Pro Asp Ser Val Cys Arg Ala Phe Val Lys Glu Ala Ala Ser Ser
   625                 630                 635                 640
   Gly Val Asp Ile Phe Arg Ile Phe Asp Ala Leu Asn Asp Val Ser Gln
                   645                 650                 655
   Met Arg Pro Ala Ile Asp Ala Val Leu Glu Thr Asn Thr Ala Val Ala
               660                 665                 670
   Glu Val Ala Met Ala Tyr Ser Gly Asp Leu Ser Asp Pro Asn Glu Lys
           675                 680                 685
   Leu Tyr Thr Leu Asp Tyr Tyr Leu Lys Met Ala Glu Glu Ile Val Lys
       690                 695                 700
   Ser Gly Ala His Ile Leu Ala Ile Lys Asp Met Ala Gly Leu Leu Arg
   705                 710                 715                 720
   Pro Ala Ala Val Thr Lys Leu Val Thr Ala Leu Arg Arg Glu Phe Asp
                   725                 730                 735
   Leu Pro Val His Val His Thr His Asp Thr Ala Gly Gly Gln Leu Ala
               740                 745                 750
   Thr Tyr Phe Ala Ala Ala Gln Ala Gly Ala Asp Ala Val Asp Gly Ala
           755                 760                 765
   Ser Ala Pro Leu Ser Gly Thr Thr Ser Gln Pro Ser Leu Ser Ala Ile
   770 775 780
   Val Ala Ala Phe Ala His Thr Arg Arg Asp Thr Gly Leu Ser Leu Glu
   785 790 795 800
   Ala Val Ser Asp Leu Glu Pro Tyr Trp Glu Ala Val Arg Gly Leu Tyr
   805 810 815
   Leu Pro Phe Glu Ser Gly Thr Pro Gly Pro Thr Gly Arg Val Tyr Arg
   820 825 830
   His Glu Ile Pro Gly Gly Gln Leu Ser Asn Leu Arg Ala Gln Ala Thr
   835 840 845
   Ala Leu Gly Leu Ala Asp Arg Phe Glu Leu Ile Glu Asp Asn Tyr Ala
   850 855 860
   Ala Val Asn Glu Met Leu Gly Arg Pro Thr Lys Val Thr Pro Ser Ser
   865 870 875 880
   Lys Val Val Gly Asp Leu Ala Leu His Leu Val Gly Ala Gly Val Asp
   885 890 895
   Pro Ala Asp Phe Ala Ala Asp Pro Gln Lys Tyr Asp Ile Pro Asp Ser
   900 905 910
   Val Ile Ala Phe Leu Arg Gly Glu Leu Gly Asn Pro Pro Gly Gly Trp
   915 920 925
   Pro Glu Pro Leu Arg Thr Arg Ala Leu Glu Gly Arg Ser Glu Gly Lys
   930 935 940
   Ala Pro Leu Thr Glu Val Pro Glu Glu Glu Gln Ala His Leu Asp Ala
   945 950 955 960
   Asp Asp Ser Lys Glu Arg Arg Asn Ser Leu Asn Arg Leu Leu Phe Pro
   965 970 975
   Lys Pro Thr Glu Glu Phe Leu Glu His Arg Arg Arg Phe Gly Asn Thr
   980 985 990
   Ser Ala Leu Asp Asp Arg Glu Phe Phe Tyr Gly Leu Val Glu Gly Arg
   995 1000 1005
   Glu Thr Leu Ser Arg Leu Pro Asp Val Arg Thr Pro Leu Leu Val
   1010 1015 1020
   Arg Leu  Asp Ala Ile Ser Glu  Pro Asp Asp Lys Gly  Met Arg Asn
       1025                 1030                 1035
   Val Val  Ala Asn Val Asn Gly  Gln Ile Arg Pro Met  Arg Val Arg
       1040                 1045                 1050
   Asp Arg  Ser Val Glu Ser Val  Thr Ala Thr Ala Glu  Lys Ala Asp
       1055                 1060                 1065
   Ser Ser  Asn Lys Gly His Val  Ala Ala Pro Phe Ala  Gly Val Val
       1070                 1075                 1080
   Thr Val  Thr Val Ala Glu Gly  Asp Glu Val Lys Ala  Gly Asp Ala
       1085                 1090                 1095
   Val Ala  Ile Ile Glu Ala Met  Lys Met Glu Ala Thr  Ile Thr Ala
       1100                 1105                 1110
   Ser Val  Asp Gly Lys Ile Asp  Arg Val Val Val Pro  Ala Ala Thr
       1115                 1120                 1125
   Lys Val  Glu Gly Gly Asp Leu  Ile Val Val Val Ser
       1130                 1135                 1140
<210> 5
   <211> 3423
   <212> DNA
   <213> Corynebacterium glutamicum
<400> 5
   atgtcgactc acacatcttc aacgcttcca gcattcaaaa agatcttggt agcaaaccgc 60
   ggcgaaatcg cggtccgtgc tttccgtgca gcactcgaaa ccggtgcagc cacggtagct 120
   atttaccccc gtgaagatcg gggatcattc caccgctctt ttgcttctga agctgtccgc 180
   attggtaccg aaggctcacc agtcaaggcg tacctggaca tcgatgaaat tatcggtgca 240
   gctaaaaaag ttaaagcaga tgccatttac ccgggatacg gcttcctgtc tgaaaatgcc 300
   cagcttgccc gcgagtgtgc ggaaaacggc attactttta ttggcccaac cccagaggtt 360
   cttgatctca ccggtgataa gtctcgcgcg gtaaccgccg cgaagaaggc tggtctgcca 420
   gttttggcgg aatccacccc gagcaaaaac atcgatgaga tcgttaaaag cgctgaaggc 480
   cagacttacc ccatctttgt gaaggcagtt gccggtggtg gcggacgcgg tatgcgtttt 540
   gttgcttcac ctgatgagct tcgcaaatta gcaacagaag catctcgtga agctgaagcg 600
   gctttcggcg atggcgcggt atatgtcgaa cgtgctgtga ttaaccctca gcatattgaa 660
   gtgcagatcc ttggcgatca cactggagaa gttgtacacc tttatgaacg tgactgctca 720
   ctgcagcgtc gtcaccaaaa agttgtcgaa attgcgccag cacagcattt ggatccagaa 780
   ctgcgtgatc gcatttgtgc ggatgcagta aagttctgcc gctccattgg ttaccagggc 840
   gcgggaaccg tggaattctt ggtcgatgaa aagggcaacc acgtcttcat cgaaatgaac 900
   ccacgtatcc aggttgagca caccgtgact gaagaagtca ccgaggtgga cctggtgaag 960
   gcgcagatgc gcttggctgc tggtgcaacc ttgaaggaat tgggtctgac ccaagataag 1020
   atcaagaccc acggtgcagc actgcagtgc cgcatcacca cggaagatcc aaacaacggc 1080
   ttccgcccag ataccggaac tatcaccgcg taccgctcac caggcggagc tggcgttcgt 1140
   cttgacggtg cagctcagct cggtggcgaa atcaccgcac actttgactc catgctggtg 1200
   aaaatgacct gccgtggttc cgactttgaa actgctgttg ctcgtgcaca gcgcgcgttg 1260
   gctgagttca ccgtgtctgg tgttgcaacc aacattggtt tcttgcgtgc gttgctgcgg 1320
   gaagaggact tcacttccaa gcgcatcgcc accggattca ttgccgatca cccgcacctc 1380
   cttcaggctc cacctgctga tgatgagcag ggacgcatcc tggattactt ggcagatgtc 1440
   accgtgaaca agcctcatgg tgtgcgtcca aaggatgttg cagctcctat cgataagctg 1500
   cctaacatca aggatctgcc actgccacgc ggttcccgtg accgcctgaa gcagcttggc 1560
   ccagccgcgt ttgctcgtga tctccgtgag caggacgcac tggcagttac tgataccacc 1620
   ttccgcgatg cacaccagtc tttgcttgcg acccgagtcc gctcattcgc actgaagcct 1680
   gcggcagagg ccgtcgcaaa gctgactcct gagcttttgt ccgtggaggc ctggggcggc 1740
   gcgacctacg atgtggcgat gcgtttcctc tttgaggatc cgtgggacag gctcgacgag 1800
   ctgcgcgagg cgatgccgaa tgtaaacatt cagatgctgc ttcgcggccg caacaccgtg 1860
   ggatacaccc cgtacccaga ctccgtctgc cgcgcgtttg ttaaggaagc tgccagctcc 1920
   ggcgtggaca tcttccgcat cttcgacgcg cttaacgacg tctcccagat gcgtccagca 1980
   atcgacgcag tcctggagac caacaccgcg gtagccgagg tggctatggc ttattctggt 2040
   gatctctctg atccaaatga aaagctctac accctggatt actacctaaa gatggcagag 2100
   gagatcgtca agtctggcgc tcacatcttg gccattaagg atatggctgg tctgcttcgc 2160
   ccagctgcgg taaccaagct ggtcaccgca ctgcgccgtg aattcgatct gccagtgcac 2220
   gtgcacaccc acgacactgc gggtggccag ctggcaacct actttgctgc agctcaagct 2280
   ggtgcagatg ctgttgacgg tgcttccgca ccactgtctg gcaccacctc ccagccatcc 2340
   ctgtctgcca ttgttgctgc attcgcgcac acccgtcgcg ataccggttt gagcctcgag 2400
   gctgtttctg acctcgagcc gtactgggaa gcagtgcgcg gactgtacct gccatttgag 2460
   tctggaaccc caggcccaac cggtcgcgtc taccgccacg aaatcccagg cggacagttg 2520
   tccaacctgc gtgcacaggc caccgcactg ggccttgcgg atcgtttcga actcatcgaa 2580
   gacaactacg cagccgttaa tgagatgctg ggacgcccaa ccaaggtcac cccatcctcc 2640
   aaggttgttg gcgacctcgc actccacctc gttggtgcgg gtgtggatcc agcagacttt 2700
   gctgccgatc cacaaaagta cgacatccca gactctgtca tcgcgttcct gcgcggcgag 2760
   cttggtaacc ctccaggtgg ctggccagag ccactgcgca cccgcgcact ggaaggccgc 2820
   tccgaaggca aggcacctct gacggaagtt cctgaggaag agcaggcgca cctcgacgct 2880
   gatgattcca aggaacgtcg caatagcctc aaccgcctgc tgttcccgaa gccaaccgaa 2940
   gagttcctcg agcaccgtcg ccgcttcggc aacacctctg cgctggatga tcgtgaattc 3000
   ttctacggcc tggtcgaagg ccgcgagact ttgatccgcc tgccagatgt gcgcacccca 3060
   ctgcttgttc gcctggatgc gatctctgag ccagacgata agggtatgcg caatgttgtg 3120
   gccaacgtca acggccagat ccgcccaatg cgtgtgcgtg accgctccgt tgagtctgtc 3180
   accgcaaccg cagaaaaggc agattcctcc aacaagggcc atgttgctgc accattcgct 3240
   ggtgttgtca ccgtgactgt tgctgaaggt gatgaggtca aggctggaga tgcagtcgca 3300
   atcatcgagg ctatgaagat ggaagcaaca atcactgctt ctgttgacgg caaaatcgat 3360
   cgcgttgtgg ttcctgctgc aacgaaggtg gaaggtggcg acttgatcgt cgtcgtttcc 3420
   taa 3423
<210> 6
   <211> 1140
   <212> PRT
   <213> Corynebacterium glutamicum
<400> 6
   Met Ser Thr His Thr Ser Ser Thr Leu Pro Ala Phe Lys Lys Ile Leu
   1               5                   10                  15
   Val Ala Asn Arg Gly Glu Ile Ala Val Arg Ala Phe Arg Ala Ala Leu
               20                  25                  30
   Glu Thr Gly Ala Ala Thr Val Ala Ile Tyr Pro Arg Glu Asp Arg Gly
           35                  40                  45
   Ser Phe His Arg Ser Phe Ala Ser Glu Ala Val Arg Ile Gly Thr Glu
       50                  55                  60
   Gly Ser Pro Val Lys Ala Tyr Leu Asp Ile Asp Glu Ile Ile Gly Ala
   65                  70                  75                  80
   Ala Lys Lys Val Lys Ala Asp Ala Ile Tyr Pro Gly Tyr Gly Phe Leu
                   85                  90                  95
   Ser Glu Asn Ala Gln Leu Ala Arg Glu Cys Ala Glu Asn Gly Ile Thr
               100                 105                 110
   Phe Ile Gly Pro Thr Pro Glu Val Leu Asp Leu Thr Gly Asp Lys Ser
           115                 120                 125
   Arg Ala Val Thr Ala Ala Lys Lys Ala Gly Leu Pro Val Leu Ala Glu
       130                 135                 140
   Ser Thr Pro Ser Lys Asn Ile Asp Glu Ile Val Lys Ser Ala Glu Gly
   145                 150                 155                 160
   Gln Thr Tyr Pro Ile Phe Val Lys Ala Val Ala Gly Gly Gly Gly Arg
                   165                 170                 175
   Gly Met Arg Phe Val Ala Ser Pro Asp Glu Leu Arg Lys Leu Ala Thr
               180                 185                 190
   Glu Ala Ser Arg Glu Ala Glu Ala Ala Phe Gly Asp Gly Ala Val Tyr
           195                 200                 205
   Val Glu Arg Ala Val Ile Asn Pro Gln His Ile Glu Val Gln Ile Leu
       210                 215                 220
   Gly Asp His Thr Gly Glu Val Val His Leu Tyr Glu Arg Asp Cys Ser
   225                 230                 235                 240
   Leu Gln Arg Arg His Gln Lys Val Val Glu Ile Ala Pro Ala Gln His
                   245                 250                 255
   Leu Asp Pro Glu Leu Arg Asp Arg Ile Cys Ala Asp Ala Val Lys Phe
               260                 265                 270
   Cys Arg Ser Ile Gly Tyr Gln Gly Ala Gly Thr Val Glu Phe Leu Val
           275                 280                 285
   Asp Glu Lys Gly Asn His Val Phe Ile Glu Met Asn Pro Arg Ile Gln
       290                 295                 300
   Val Glu His Thr Val Thr Glu Glu Val Thr Glu Val Asp Leu Val Lys
   305                 310                 315                 320
   Ala Gln Met Arg Leu Ala Ala Gly Ala Thr Leu Lys Glu Leu Gly Leu
                   325                 330                 335
   Thr Gln Asp Lys Ile Lys Thr His Gly Ala Ala Leu Gln Cys Arg Ile
               340                 345                 350
   Thr Thr Glu Asp Pro Asn Asn Gly Phe Arg Pro Asp Thr Gly Thr Ile
           355                 360                 365
   Thr Ala Tyr Arg Ser Pro Gly Gly Ala Gly Val Arg Leu Asp Gly Ala
   370 375 380
   Ala Gln Leu Gly Gly Glu Ile Thr Ala His Phe Asp Ser Met Leu Val
   385 390 395 400
   Lys Met Thr Cys Arg Gly Ser Asp Phe Glu Thr Ala Val Ala Arg Ala
   405 410 415
   Gln Arg Ala Leu Ala Glu Phe Thr Val Ser Gly Val Ala Thr Asn Ile
   420 425 430
   Gly Phe Leu Arg Ala Leu Leu Arg Glu Glu Asp Phe Thr Ser Lys Arg
   435 440 445
   Ile Ala Thr Gly Phe Ile Ala Asp His Pro His Leu Leu Gln Ala Pro
   450 455 460
   Pro Ala Asp Asp Glu Gln Gly Arg Ile Leu Asp Tyr Leu Ala Asp Val
   465 470 475 480
   Thr Val Asn Lys Pro His Gly Val Arg Pro Lys Asp Val Ala Ala Pro
   485 490 495
   Ile Asp Lys Leu Pro Asn Ile Lys Asp Leu Pro Leu Pro Arg Gly Ser
   500 505 510
   Arg Asp Arg Leu Lys Gln Leu Gly Pro Ala Ala Phe Ala Arg Asp Leu
   515 520 525
   Arg Glu Gln Asp Ala Leu Ala Val Thr Asp Thr Thr Phe Arg Asp Ala
   530 535 540
   His Gln Ser Leu Leu Ala Thr Arg Val Arg Ser Phe Ala Leu Lys Pro
   545 550 555 560
   Ala Ala Glu Ala Val Ala Lys Leu Thr Pro Glu Leu Leu Ser Val Glu
   565 570 575
   Ala Trp Gly Gly Ala Thr Tyr Asp Val Ala Met Arg Phe Leu Phe Glu
   580 585 590
   Asp Pro Trp Asp Arg Leu Asp Glu Leu Arg Glu Ala Met Pro Asn Val
   595 600 605
   Asn Ile Gln Met Leu Leu Arg Gly Arg Asn Thr Val Gly Tyr Thr Pro
       610                 615                 620
   Tyr Pro Asp Ser Val Cys Arg Ala Phe Val Lys Glu Ala Ala Ser Ser
   625                 630                 635                 640
   Gly Val Asp Ile Phe Arg Ile Phe Asp Ala Leu Asn Asp Val Ser Gln
                   645                 650                 655
   Met Arg Pro Ala Ile Asp Ala Val Leu Glu Thr Asn Thr Ala Val Ala
               660                 665                 670
   Glu Val Ala Met Ala Tyr Ser Gly Asp Leu Ser Asp Pro Asn Glu Lys
           675                 680                 685
   Leu Tyr Thr Leu Asp Tyr Tyr Leu Lys Met Ala Glu Glu Ile Val Lys
       690                 695                 700
   Ser Gly Ala His Ile Leu Ala Ile Lys Asp Met Ala Gly Leu Leu Arg
   705                 710                 715                 720
   Pro Ala Ala Val Thr Lys Leu Val Thr Ala Leu Arg Arg Glu Phe Asp
                   725                 730                 735
   Leu Pro Val His Val His Thr His Asp Thr Ala Gly Gly Gln Leu Ala
               740                 745                 750
   Thr Tyr Phe Ala Ala Ala Gln Ala Gly Ala Asp Ala Val Asp Gly Ala
           755                 760                 765
   Ser Ala Pro Leu Ser Gly Thr Thr Ser Gln Pro Ser Leu Ser Ala Ile
       770                 775                 780
   Val Ala Ala Phe Ala His Thr Arg Arg Asp Thr Gly Leu Ser Leu Glu
   785                 790                 795                 800
   Ala Val Ser Asp Leu Glu Pro Tyr Trp Glu Ala Val Arg Gly Leu Tyr
                   805                 810                 815
   Leu Pro Phe Glu Ser Gly Thr Pro Gly Pro Thr Gly Arg Val Tyr Arg
               820                 825                 830
   His Glu Ile Pro Gly Gly Gln Leu Ser Asn Leu Arg Ala Gln Ala Thr
           835                 840                 845
   Ala Leu Gly Leu Ala Asp Arg Phe Glu Leu Ile Glu Asp Asn Tyr Ala
       850                 855                 860
   Ala Val Asn Glu Met Leu Gly Arg Pro Thr Lys Val Thr Pro Ser Ser
   865                 870                 875                 880
   Lys Val Val Gly Asp Leu Ala Leu His Leu Val Gly Ala Gly Val Asp
                   885                 890                 895
   Pro Ala Asp Phe Ala Ala Asp Pro Gln Lys Tyr Asp Ile Pro Asp Ser
               900                 905                 910
   Val Ile Ala Phe Leu Arg Gly Glu Leu Gly Asn Pro Pro Gly Gly Trp
           915                 920                 925
   Pro Glu Pro Leu Arg Thr Arg Ala Leu Glu Gly Arg Ser Glu Gly Lys
       930                 935                 940
   Ala Pro Leu Thr Glu Val Pro Glu Glu Glu Gln Ala His Leu Asp Ala
   945                 950                 955                 960
   Asp Asp Ser Lys Glu Arg Arg Asn Ser Leu Asn Arg Leu Leu Phe Pro
                   965                 970                 975
   Lys Pro Thr Glu Glu Phe Leu Glu His Arg Arg Arg Phe Gly Asn Thr
               980                 985                 990
   Ser Ala Leu Asp Asp Arg Glu Phe  Phe Tyr Gly Leu Val  Glu Gly Arg
           995                 1000                1005
   Glu Thr  Leu Ile Arg Leu Pro  Asp Val Arg Thr Pro  Leu Leu Val
       1010                 1015                 1020
   Arg Leu  Asp Ala Ile Ser Glu  Pro Asp Asp Lys Gly  Met Arg Asn
       1025                 1030                 1035
   Val Val  Ala Asn Val Asn Gly  Gln Ile Arg Pro Met  Arg Val Arg
       1040                 1045                 1050
   Asp Arg  Ser Val Glu Ser Val  Thr Ala Thr Ala Glu  Lys Ala Asp
       1055                 1060                 1065
   Ser Ser  Asn Lys Gly His Val  Ala Ala Pro Phe Ala  Gly Val Val
       1070                 1075                 1080
   Thr Val  Thr Val Ala Glu Gly  Asp Glu Val Lys Ala  Gly Asp Ala
       1085                 1090                 1095
   Val Ala  Ile Ile Glu Ala Met  Lys Met Glu Ala Thr  Ile Thr Ala
       1100                 1105                 1110
   Ser Val  Asp Gly Lys Ile Asp  Arg Val Val Val Pro  Ala Ala Thr
       1115                 1120                 1125
   Lys Val  Glu Gly Gly Asp Leu  Ile Val Val Val Ser
       1130                 1135                 1140
<210> 7
   <211> 3423
   <212> DNA
   <213> Corynebacterium glutamicum
<400> 7
   atgtcgactc acacatcttc aacgcttcca gcattcaaaa agatcttggt agcaaaccgc      60
   ggcgaaatcg cggtccgtgc tttccgtgca gcactcgaaa ccggtgcagc cacggtagct     120
   atttaccccc gtgaagatcg gggatcattc caccgctctt ttgcttctga agctgtccgc     180
   attggtaccg aaggctcacc agtcaaggcg tacctggaca tcgatgaaat tatcggtgca     240
   gctaaaaaag ttaaagcaga tgccatttac ccgggatacg gcttcctgtc tgaaaatgcc     300
   cagcttgccc gcgagtgtgc ggaaaacggc attactttta ttggcccaac cccagaggtt     360
   cttgatctca ccggtgataa gtctcgcgcg gtaaccgccg cgaagaaggc tggtctgcca     420
   gttttggcgg aatccacccc gagcaaaaac atcgatgaga tcgttaaaag cgctgaaggc     480
   cagacttacc ccatctttgt gaaggcagtt gccggtggtg gcggacgcgg tatgcgtttt     540
   gttgcttcac ctgatgagct tcgcaaatta gcaacagaag catctcgtga agctgaagcg     600
   gctttcggcg atggcgcggt atatgtcgaa cgtgctgtga ttaaccctca gcatattgaa     660
   gtgcagatcc ttggcgatca cactggagaa gttgtacacc tttatgaacg tgactgctca     720
   ctgcagcgtc gtcaccaaaa agttgtcgaa attgcgccag cacagcattt ggatccagaa     780
   ctgcgtgatc gcatttgtgc ggatgcagta aagttctgcc gctccattgg ttaccagggc     840
   gcgggaaccg tggaattctt ggtcgatgaa aagggcaacc acgtcttcat cgaaatgaac     900
   ccacgtatcc aggttgagca caccgtgact gaagaagtca ccgaggtgga cctggtgaag     960
   gcgcagatgc gcttggctgc tggtgcaacc ttgaaggaat tgggtctgac ccaagataag    1020
   atcaaggccc acggggcagc actgcagtgc cgcatcacca cggaagatcc aaacaacggc    1080
   ttccgcccag ataccggaac tatcaccgcg taccgctcac caggcggagc tggcgttcgt    1140
   cttgacggtg cagctcagct cggtggcgaa atcaccgcac actttgactc catgctggtg    1200
   aaaatgacct gccgtggttc cgactttgaa actgctgttg ctcgtgcaca gcgcgcgttg    1260
   gctgagttca ccgtgtctgg tgttgcaacc aacattggtt tcttgcgtgc gttgctgcgg    1320
   gaagaggact tcacttccaa gcgcatcgcc accggattca ttgccgatca cccgcacctc    1380
   cttcaggctc cacctgctga tgatgagcag ggacgcatcc tggattactt ggcagatgtc    1440
   accgtgaaca agcctcatgg tgtgcgtcca aaggatgttg cagctcctat cgataagctg 1500
   cctaacatca aggatctgcc actgccacgc ggttcccgtg accgcctgaa gcagcttggc 1560
   ccagccgcgt ttgctcgtga tctccgtgag caggacgcac tggcagttac tgataccacc 1620
   ttccgcgatg cacaccagtc tttgcttgcg acccgagtcc gctcattcgc actgaagcct 1680
   gcggcagagg ccgtcgcaaa gctgactcct gagcttttgt ccgtggaggc ctggggcggc 1740
   gcgacctacg atgtggcgat gcgtttcctc tttgaggatc cgtgggacag gctcgacgag 1800
   ctgcgcgagg cgatgccgaa tgtaaacatt cagatgctgc ttcgcggccg caacaccgtg 1860
   ggatacaccc cgtacccaga ctccgtctgc cgcgcgtttg ttaaggaagc tgccagctcc 1920
   ggcgtggaca tcttccgcat cttcgacgcg cttaacgacg tctcccagat gcgtccagca 1980
   atcgacgcag tcctggagac caacaccgcg gtagccgagg tggctatggc ttattctggt 2040
   gatctctctg atccaaatga aaagctctac accctggatt actacctaaa gatggcagag 2100
   gagatcgtca agtctggcgc tcacatcttg gccattaagg atatggctgg tctgcttcgc 2160
   ccagctgcgg taaccaagct ggtcaccgca ctgcgccgtg aattcgatct gccagtgcac 2220
   gtgcacaccc acgacactgc gggtggccag ctggcaacct actttgctgc agctcaagct 2280
   ggtgcagatg ctgttgacgg tgcttccgca ccactgtctg gcaccacctc ccagccatcc 2340
   ctgtctgcca ttgttgctgc attcgcgcac acccgtcgcg ataccggttt gagcctcgag 2400
   gctgtttctg acctcgagcc gtactgggaa gcagtgcgcg gactgtacct gccatttgag 2460
   tctggaaccc caggcccaac cggtcgcgtc taccgccacg aaatcccagg cggacagttg 2520
   tccaacctgc gtgcacaggc caccgcactg ggccttgcgg atcgtttcga actcatcgaa 2580
   gacaactacg cagccgttaa tgagatgctg ggacgcccaa ccaaggtcac cccatcctcc 2640
   aaggttgttg gcgacctcgc actccacctc gttggtgcgg gtgtggatcc agcagacttt 2700
   gctgccgatc cacaaaagta cgacatccca gactctgtca tcgcgttcct gcgcggcgag 2760
   cttggtaacc ctccaggtgg ctggccagag ccactgcgca cccgcgcact ggaaggccgc 2820
   tccgaaggca aggcacctct gacggaagtt cctgaggaag agcaggcgca cctcgacgct 2880
   gatgattcca aggaacgtcg caatagcctc aaccgcctgc tgttcccgaa gccaaccgaa 2940
   gagttcctcg agcaccgtcg ccgcttcggc aacacctctg cgctggatga tcgtgaattc 3000
   ttctacggcc tggtcgaagg ccgcgagact ttgatccgcc tgccagatgt gcgcacccca 3060
   ctgcttgttc gcctggatgc gatctctgag ccagacgata agggtatgcg caatgttgtg 3120
   gccaacgtca acggccagat ccgcccaatg cgtgtgcgtg accgctccgt tgagtctgtc 3180
   accgcaaccg cagaaaaggc agattcctcc aacaagggcc atgttgctgc accattcgct 3240
   ggtgttgtca ccgtgactgt tgctgaaggt gatgaggtca aggctggaga tgcagtcgca 3300
   atcatcgagg ctatgaagat ggaagcaaca atcactgctt ctgttgacgg caaaatcgat 3360
   cgcgttgtgg ttcctgctgc aacgaaggtg gaaggtggcg acttgatcgt cgtcgtttcc 3420
   taa 3423
<210> 8
   <211> 3423
   <212> DNA
   <213> Corynebacterium glutamicum
<400> 8
   atgtcgactc acacatcttc aacgcttcca gcattcaaaa agatcttggt agcaaaccgc      60
   ggcgaaatcg cggtccgtgc tttccgtgca gcactcgaaa ccggtgcagc cacggtagct     120
   atttaccccc gtgaagatcg gggatcattc caccgctctt ttgcttctga agctgtccgc     180
   attggtaccg aaggctcacc agtcaaggcg tacctggaca tcgatgaaat tatcggtgca     240
   gctaaaaaag ttaaagcaga tgccatttac ccgggatacg gcttcctgtc tgaaaatgcc     300
   cagcttgccc gcgagtgtgc ggaaaacggc attactttta ttggcccaac cccagaggtt     360
   cttgatctca ccggtgataa gtctcgcgcg gtaaccgccg cgaagaaggc tggtctgcca     420
   gttttggcgg aatccacccc gagcaaaaac atcgatgaga tcgttaaaag cgctgaaggc     480
   cagacttacc ccatctttgt gaaggcagtt gccggtggtg gcggacgcgg tatgcgtttt     540
   gttgcttcac ctgatgagct tcgcaaatta gcaacagaag catctcgtga agctgaagcg     600
   gctttcggcg atggcgcggt atatgtcgaa cgtgctgtga ttaaccctca gcatattgaa     660
   gtgcagatcc ttggcgatca cactggagaa gttgtacacc tttatgaacg tgactgctca     720
   ctgcagcgtc gtcaccaaaa agttgtcgaa attgcgccag cacagcattt ggatccagaa     780
   ctgcgtgatc gcatttgtgc ggatgcagta aagttctgcc gctccattgg ttaccagggc     840
   gcgggaaccg tggaattctt ggtcgatgaa aagggcaacc acgtcttcat cgaaatgaac     900
   ccacgtatcc aggttgagca caccgtgact gaagaagtca ccgaggtgga cctggtgaag     960
   gcgcagatgc gcttggctgc tggtgcaacc ttgaaggaat tgggtctgac ccaagataag    1020
   atcaaggccc acggggcagc actgcagtgc cgcatcacca cggaagatcc aaacaacggc    1080
   ttccgcccag ataccggaac tatcaccgcg taccgctcac caggcggagc tggcgttcgt    1140
   cttgacggtg cagctcagct cggtggcgaa atcaccgcac actttgactc catgctggtg    1200
   aaaatgacct gccgtggttc cgactttgaa actgctgttg ctcgtgcaca gcgcgcgttg    1260
   gctgagttca ccgtgtctgg tgttgcaacc aacattggtt tcttgcgtgc gttgctgcgg    1320
   gaagaggact tcacttccaa gcgcatcgcc accggattca ttgccgatca cccgcacctc    1380
   cttcaggctc cacctgctga tgatgagcag ggacgcatcc tggattactt ggcagatgtc    1440
   accgtgaaca agcctcatgg tgtgcgtcca aaggatgttg cagctcctat cgataagctg    1500
   cctaacatca aggatctgcc actgccacgc ggttcccgtg accgcctgaa gcagcttggc    1560
   ccagccgcgt ttgctcgtga tctccgtgag caggacgcac tggcagttac tgataccacc 1620
   ttccgcgatg cacaccagtc tttgcttgcg acccgagtcc gctcattcgc actgaagcct 1680
   gcggcagagg ccgtcgcaaa gctgactcct gagcttttgt ccgtggaggc ctggggcggc 1740
   gcgacctacg atgtggcgat gcgtttcctc tttgaggatc cgtgggacag gctcgacgag 1800
   ctgcgcgagg cgatgccgaa tgtaaacatt cagatgctgc ttcgcggccg caacaccgtg 1860
   ggatacaccc cgtacccaga ctccgtctgc cgcgcgtttg ttaaggaagc tgccagctcc 1920
   ggcgtggaca tcttccgcat cttcgacgcg cttaacgacg tctcccagat gcgtccagca 1980
   atcgacgcag tcctggagac caacaccgcg gtagccgagg tggctatggc ttattctggt 2040
   gatctctctg atccaaatga aaagctctac accctggatt actacctaaa gatggcagag 2100
   gagatcgtca agtctggcgc tcacatcttg gccattaagg atatggctgg tctgcttcgc 2160
   ccagctgcgg taaccaagct ggtcaccgca ctgcgccgtg aattcgatct gccagtgcac 2220
   gtgcacaccc acgacactgc gggtggccag ctggcaacct actttgctgc agctcaagct 2280
   ggtgcagatg ctgttgacgg tgcttccgca ccactgtctg gcaccacctc ccagccatcc 2340
   ctgtctgcca ttgttgctgc attcgcgcac acccgtcgcg ataccggttt gagcctcgag 2400
   gctgtttctg acctcgagcc gtactgggaa gcagtgcgcg gactgtacct gccatttgag 2460
   tctggaaccc caggcccaac cggtcgcgtc taccgccacg aaatcccagg cggacagttg 2520
   tccaacctgc gtgcacaggc caccgcactg ggccttgcgg atcgtttcga actcatcgaa 2580
   gacaactacg cagccgttaa tgagatgctg ggacgcccaa ccaaggtcac cccatcctcc 2640
   aaggttgttg gcgacctcgc actccacctc gttggtgcgg gtgtggatcc agcagacttt 2700
   gctgccgatc cacaaaagta cgacatccca gactctgtca tcgcgttcct gcgcggcgag 2760
   cttggtaacc ctccaggtgg ctggccagag ccactgcgca cccgcgcact ggaaggccgc 2820
   tccgaaggca aggcacctct gacggaagtt cctgaggaag agcaggcgca cctcgacgct 2880
   gatgattcca aggaacgtcg caatagcctc aaccgcctgc tgttcccgaa gccaaccgaa 2940
   gagttcctcg agcaccgtcg ccgcttcggc aacacctctg cgctggatga tcgtgaattc 3000
   ttctacggcc tggtcgaagg ccgcgagact ttgagccggc tgccagatgt gcgcacccca 3060
   ctgcttgttc gcctggatgc gatctctgag ccagacgata agggtatgcg caatgttgtg 3120
   gccaacgtca acggccagat ccgcccaatg cgtgtgcgtg accgctccgt tgagtctgtc 3180
   accgcaaccg cagaaaaggc agattcctcc aacaagggcc atgttgctgc accattcgct 3240
   ggtgttgtca ccgtgactgt tgctgaaggt gatgaggtca aggctggaga tgcagtcgca 3300
   atcatcgagg ctatgaagat ggaagcaaca atcactgctt ctgttgacgg caaaatcgat 3360
   cgcgttgtgg ttcctgctgc aacgaaggtg gaaggtggcg acttgatcgt cgtcgtttcc 3420
   taa 3423

## Beschreibung

Die Erfindung betrifft eine Pyruvatcarboxylase und eine für die Pyruvatcarboxylase kodierende DNA, ein Plasmid enthaltend die DNA sowie einen Mikroorganismus zur Produktion und ein Verfahren zur Herstellung von Produkten, deren Biosynthese Oxalacetat als Vorstufe beinhalten und ein Chromosom.

*Corynebacterium glutamicum* wird industriell für die Herstellung von Aminosäuren, insbesondere L-Glutamat und L-Lysin, genutzt. Für die Produktion von L-Lysin wird dem Citrat-Zyklus das Intermediat Oxalacetat entzogen, da es als Vorstufe für Aminosäuren oder Salze der Aminosäuren der Aspartatfamilie dient. Diese sind L-Aspartat, L-Asparagin, L-Lysin, L-Methionin, L-Threonin und L-Isoleucin. Damit der Citrat-Zyklus trotz des Entzugs dieser Intermediate weiter ablaufen kann, muss er durch sogenannte anaplerotische Reaktionen wieder aufgefüllt werden. Bei Wachstum auf Zuckern erfolgt das Auffüllen des Oxalacetat-Pools durch die Carboxylierung von Pyruvat oder Phosphoenolpyruvat zu Oxalacetat. Die ATP-abhängige Bildung von Oxalacetat aus Pyruvat und Kohlenstoffdioxid bzw. HCO₃⁻ wird durch das Enzym Pyruvatcarboxylase (Pyc) katalysiert. Für die industrielle mikrobielle Produktion von Aminosäuren der Aspartat-Familie wie z.B. L-Lysin und der Glutamat-Familie wie z.B. L-Glutamat ist demnach eine hohe Aktivität der Pyc wichtig. Auch für die Produktion anderer Metabolite, die sich aus Intermediaten des Citratzyklus ableiten, ist eine hohe Pyc-Aktivität förderlich.

Die enzymatische Aktivität der nativen Pyc von *C*. *glutamicum* wird allosterisch unter anderem durch Aspartat gehemmt und hat daher bei hohen intrazellulären Aspartat-Konzentrationen nur eine begrenzte Aktivität. Damit ist auch die Produktion von L-Lysin und anderen, vom Oxalacetat abgeleiteten Produkten, begrenzt, da nur eine begrenzte Menge der Vorläufer-Metabolite bereitgestellt wird. Die bisher beschriebenen Pyc-Varianten führen nur zu einer moderaten Erhöhung der L-Lysinproduktion.

Die Veröffentlichung von Peters-Wendisch et al. "Pyruvate carboxylase is a major bottleneck for glutamate and lysine production by Corynebacterium glutamicum" im Journal of Molecular Microbiology and Biotechnology (2001) 32: 295-300 offenbart, dass die Deletion des *pyc-*Gens im Stamm *C. glutamicum* DG52-5 zu einer 60%igen Reduktion des Lysin-Titers führt, während die plasmid-vermittelte Überexpression des pyc-Gens im Stamm DG52-5 zu einem um 50% gesteigerten Lysin-Titer führt. Weiterhin wird gezeigt, dass die Deletion des *pyc-*Gens im Wildtyp-Stamm ATCC13032 die durch Tween 60 induzierte L-Glutamat-Produktion um etwa 50% reduziert, während die plasmid-basierte Überexpression des *pyc*-Gens die Glutamat-Bildung um 700% steigert. Die Wichtigkeit einer hohen Pyc-Aktivität für die Produktion von Lysin und Glutamat wurde später auch noch in anderen Publikationen gezeigt (Blombach et al. 2007 Applied Microbiology and Biotechnology 76: 615-623; Shirai et al. 2007 Microbial Cell Factories 6: 19; Neuner und Heinzle 2011 Biotechnology Journal 6: 318-329; Neuner et al. 2013 Journal of Biotechnology 163: 217-224; Guo et al. 2013 Biotechnology Letters 35: 943-950).

Auch für die Produktion anderer Metabolite außer Aminosäuren, die Intermediate des Citratzyklus darstellen oder sich von Intermediaten des Citratzyklus ableiten, ist eine hohe Pyc-Aktivität von großer Bedeutung. Die Veröffentlichungen von Shohei Okino et al. "An efficient succinic acid production process in a metabolically engineered Corynebacterium glutamicum strain" in Appl. Microbiol. Biotechnol. (2008) 81: 459-464 und Torben Hoefel et al. "Comparative reaction engineering studies for succinic acid production from sucrose by metabolically engineered Escherichia coli in fed-batch-operated stirred tank bioreactiors" in Biotechnol. J. 2012, 7, 1277-1287 offenbaren, dass die Expression bzw. Überexpression von Genen, kodierend für eine Pyruvatcarboxylase, zu einer Produktion oder gesteigerten Produktion von Succinat führt. Dies wird auch in weiteren Publikationen gezeigt (Li et al. 2016 Bioresource Technology 218: 217-223; Tajima et al. 2015 Applied and Environmental Microbiology 81: 929-937; Litsanov et al. 2012 Applied and Environmental Microbiology 78: 3325-3337; Meng et al. 2016 Microbial Cell Factories 15: 141). Für die Produktion von Malat mit *Thermobifida fusca* erwies sich eine erhöhte Pyc-Aktivität ebenfalls als förderlich (Deng et al. 2016 Biotechnology Progress 31: 14-20). Ebenfalls wurde für die Produktion von Diaminen, die sich aus Intermediaten des Citratzyklus ableiten, wie z.B. Putrescin (1,4-Diaminobutan) oder Cadaverin (1,5-Diaminopentan) eine Verstärkung der Pyc-Aktvität eingesetzt (Nguyen et al. 2015 Metabolites 5: 211-231; Kind et al. 2010 Metabolic Engineering 12: 341-351).

Ohnishi et al. (Applied Microbiology and Biotechnology (2002) 58: 217-223) haben die chromosomale Einführung des Aminosäureaustausches Prolin zu Serin an Position 458 der Pyc von C. *glutamicum* in den *C. glutamicum*-Stamm AHD2 beschrieben, der auf dem Wildtyp ATCC 13032 basiert und zwei Punktmutationen trägt, Val59Ala im Gen für die Homoserin-Dehydrogenase (hom) und Thr311 Ile im Gen für die Aspartatkinase (*lysC*). Der Stamm AHP-3 mit der pyc-P458S-Mutation bildete 6% mehr L-Lysin als der Parentalstamm AHD-2. Die Autoren beschreiben, dass es für die Identifizierung der pyc-Mutation keinen bekannten selektierbaren Phänotyp gibt und sie vermutlich nur durch vergleichende Genomanalyse zu finden sei. Darüber hinaus wurde die Pyc-Variante Pyc-P458S noch nicht weiter charakterisiert.

Die Schrift US 7,300,777 B2 beschreibt einen Organismus, in welchem eine Pyruvatcarboxylase aus dem *C. glutamicum*-Stamm NRRL-B11474 mit mehreren Mutationen (M1V, E153D, A182S, A206S, H227R, A452G, D1120E) gegenüber der Pyc aus dem *C*. *glutamicum*-Stamm ATCC 21523 isoliert wurde. Mindestens eine der genannten Mutationen führt zu einer Pyc-Variante, die durch Aspartat in niedrigen Konzentrationen (1-10 mM) bis zu 2,5-fach in ihrer Aktivität stimuliert wird und bei höheren Aspartat-Konzentrationen wieder inhibiert wird bis zu maximal 30% der Aktivität in Abwesenheit von Aspartat. Bei 30 mM Aspartat zeigte die Pyc aus Stamm NRRL-B11474 noch die gleiche Aktivität wie in Abwesenheit von Aspartat, während die Pyc des Stammes ATCC 21523 bei 30 mM Aspartat nur noch 30% der Aktivität besaß, die sie in Abwesenheit von Aspartat zeigte. Die Auswirkung der feedback-resistenten Pyc-Variante aus C. *glutamicum* NRRL-B11474 auf die fermentative Produktion von Aminosäuren, insbesondere L-Lysin und L-Glutamat, wurde in der Schrift US 7,300,777 B2 jedoch nicht offenbart.

Die Deutsche Patentanmeldung 102012016716.4 offenbart ein Screening -Verfahren, mit dem verbesserte Enzyme aufgefunden werden können.

Es ist daher die Aufgabe der Erfindung, einen Mikroorganismus, eine Pyruvatcarboxylase, ein für die Pyruvatcarboxylase kodierendes Gen, ein Plasmid oder Chromosom enthaltend dieses Gen sowie ein Verfahren zur Herstellung von Produkten, deren Biosynthese Oxalacetat als Vorstufe beinhaltet, zur Verfügung zu stellen, mit denen die Ausbeute, der Titer, die volumetrische Produktivität (g Produkt/Liter/Stunde) oder die spezifische Produktivität (g Produkt/Stunde/g Zelltrockenmasse) bei der Produktion von aus Oxalacetat abgeleiteten Produkten gesteigert werden kann. Insbesondere soll die Produktion von Aminosäuren der Aspartat-Familie gesteigert werden, also L-Lysin, L-Aspartat, L-Asparagin, L-Threonin, L-Isoleucin und L-Methionin. Weiterhin soll die Produktion von Aminosäuren der Glutamat-Familie, wie L-Glutamat, L-Glutamin, L-Arginin oder L-Prolin, von Intermediaten wie Salzen und Säuren des Citrat-Zyklus beispielsweise Succinat, Malat, Fumarat, 2-Oxoglutarat, Citrat oder Isocitrat, von Diaminen wie zum Beispiel 1,5-Diaminopentan oder 1,4-Diaminobutan oder auch von weiteren Produkten wie Itaconat, Ectoin, gamma-Aminobutyrat, Butanol, 1-Propanol, L-Citrullin, L-Ornithin, D-Arginin oder 4-Hydroxyprolin gesteigert werden.

Ausgehend vom Oberbegriff des Anspruchs 1 und den nebengeordneten Ansprüchen wird die Aufgabe erfindungsgemäß gelöst mit den im kennzeichnenden Teil des Anspruchs 1 und der nebengeordneten Ansprüche angegebenen Merkmalen.

Mit dem Mikroorganismus, der Pyruvatcarboxylase, dem für die Pyruvatcarboxylase kodierenden Gen, dem Plasmid oder Chromosom enthaltend dieses Gen, sowie mit dem Herstellungsverfahren kann die Ausbeute von Produkten, deren Biosynthese Oxalacetat als Vorstufe beinhalten, gesteigert werden. Insbesondere kann die Produktion von Aminosäuren der Oxalacetat/Aspartat-Familie gesteigert werden, also L-Lysin, L-Aspartat, L-Asparagin, L-Threonin, L-Isoleucin, L-Methionin. Weiterhin kann die Produktion von Aminosäuren der Glutamat-Familie wie L-Glutamat, L-Glutamin, L-Arginin oder L-Prolin, von Intermediaten wie Salzen und Säuren des Citrat-Zyklus, beispielsweise Succinat, Malat, Fumarat oder 2-Oxoglutarat, Citrat oder Isocitrat, von Diaminen beispielsweise. 1,5-Diaminopentan oder 1,4-Diaminobutan oder auch von weiteren Produkten wie Itaconat, Ectoin, gamma-Aminobutyrat, Butanol, 1-Propanol, L-Citrullin, L-Ornithin, D-Arginin oder 4-Hydroxyprolin gesteigert werden. Vorteilhafte Weiterbildungen der Erfindung sind in den Unteransprüchen angegeben.

Im Folgenden wird die Erfindung in ihrer allgemeinen Form beschrieben, ohne dass dies einschränkend auszulegen ist.

Überraschenderweise wurde gefunden, dass eine gegenüber dem Stamm *C*. *glutamicum* ATCC 13032 *lysC*^{T311I} veränderte Pyruvatcarboxylase mit einem Austausch von Threonin in Position 343 durch Alanin, und ein für diese Pyruvatcarboxylase kodierendes, genetisch verändertes Gen, ein Plasmid, enthaltend dieses Gen, sowie ein Mikroorganismus enthaltend dieses Gen oder Plasmid sowie das Herstellungsverfahren die gestellten Aufgaben löst. Beispielsweise wird für die Produktion von L-Lysin gegenüber dem Stamm ATCC 13032 *lysC*^{T311I} eine um 15% gesteigerte Endkonzentration an L-Lysin festgestellt.

In einer bevorzugten Variante beinhaltet eine gegenüber dem Stamm C. *glutamicum* ATCC 13032 *lysC*^{T311I} veränderte Pyruvatcarboxylase neben dem Austausch von Threonin in Position 343 durch Alanin zusätzlich einen Austausch vom Isoleucin in Position 1012 durch Serin. Entsprechend betrifft die Erfindung auch ein für diese Pyruvatcarboxylase mit den Veränderungen T343A und I1012S kodierendes, genetisch verändertes Gen, ein Plasmid, enthaltend dieses Gen, sowie einen Mikroorganismus enthaltend dieses Gen oder Plasmid sowie das Herstellungsverfahren. In dieser Ausführungsform wird für die Produktion von L-Lysin gegenüber dem Stamm ATCC 13032 *lysC*^{T311I} eine um 9 bis 19% gesteigerte Endkonzentration an L-Lysin festgestellt.

Erfindungsgemäß wird ein Gen einer Identität von mindestens 70% des Gens nach Sequenz Nr.1, kodierend für eine Pyruvatcarboxylase zur Verfügung gestellt, welches ausgehend von dem Gen einer mindestens 70%igen Identität von Sequenz Nr.1 in Position 1027-1029 einen Austausch des für Threonin-343 kodierenden Tripletts gegen ein Triplett kodierend für Alanin besitzt. Position 1027-1029 kodiert daher für Alanin. Von der erfindungsgemäßen DNA sind Sequenzen einer 70%igen bis 100%igen Identität umfasst. Vorzugsweise ist die Identität 80%, 85% bis 90%. Besonders bevorzugt 91%, 92%, 93%, 94%, 95%, 96%, 97%, 98% oder 99%. Ganz besonders bevorzugt ist das Gen nach Sequenz Nr. 1.

In einer bevorzugten Ausgestaltung wird ein Gen einer Identität von mindestens 70% des Gens nach Sequenz Nr.1, kodierend für eine Pyruvatcarboxylase zur Verfügung gestellt, welches ausgehend von dem Gen einer mindestens 70%igen Identität von Sequenz Nr.1 zusätzlich in der Position 3034-3036 ein Triplett besitzt, welches für Serin kodiert. Auch in dieser Ausführungsform sind Sequenzen einer 70%igen bis 100%igen Identität umfasst. Vorzugsweise ist die Identität 80%, 85% bis 90%. Besonders bevorzugt 91%, 92%, 93%, 94%, 95%, 96%, 97%, 98% oder 99%. Ganz besonders bevorzugt ist das Gen nach Sequenz Nr. 2

Identität wie hierin verwendet, kann durch die Gleichung I (%) = [1-V/X] × 100, definiert werden, worin I Identität bedeutet, X die Gesamtzahl an Nukleobasen der Vergleichssequenz ist und V die Anzahl an unterschiedlichen Nukleobasen der zu betrachtenden Sequenz bezogen auf die Vergleichssequenz ist. Auf jeden Fall sind mit dem Begriff Nukleinsäuresequenzen, welche für Polypeptide kodieren, alle Sequenzen umfasst, die nach Maßgabe der Degeneration des genetischen Codes möglich erscheinen.

Weiterhin wird erfindungsgemäß ein Vektor, vorzugsweise ein Plasmid, enthaltend dieses Gen kodierend für eine Pyruvatcarboxylase mit der Veränderung T343A oder T343A und I1012S zur Verfügung gestellt. Dabei kommt grundsätzlich jeder Leervektor oder jedes Leerplasmid als Ausgangsvektor oder Ausgangsplasmid in Betracht. Beispielsweise kann als Leervektor das Plasmid pAN6, wie es in der Veröffentlichung von Frunzke et al., Molecular Microbiology 2008, 67:305-322 beschrieben wird, eingesetzt werden, bei dem das erfindungsgemäße Gen inseriert ist.

Erfindungsgemäß wird auch ein Chromosom zur Verfügung gestellt, welches die erfindungsgemäße DNA mit der Veränderung T343A oder T343A und I1012S enthält. Dabei soll die erfindungsgemäße DNA so in das Chromosom inseriert sein, dass die Funktion der für die Lebensfähigkeit des Mikroorganismus relevanten Gene nicht beeinträchtigt oder zerstört wird.

Durch Expression des Gens nach Sequenz Nr. 1 wird eine erfindungsgemäße Pyruvatcarboxylase mit einer mindestens 90%igen Sequenzidentität zu der Pyruvatcarboxylase nach Sequenz Nr. 3 erhalten, bei welcher ausgehend von der Pyruvatcarboxylase des Stammes ATCC 13032 *lysC*^{T311I} das Threonin an Position 343 durch Alanin ersetzt ist (*pyc*^{T343A}). In Position 343 der Pyruvatcarboxylase befindet sich daher erfindungsgemäß Alanin, wie beispielsweise in Seq. Nr.3. Von der erfindungsgemäßen Pyruvatcarboxylase sind Pyruvatcarboxylasen einer 90%igen bis 100%igen Identität zu der Sequenz Nr. 3 umfasst. Vorzugsweise ist die Identität 95%, 96%, oder 97%, besonders bevorzugt 98% oder 99% der erfindungsgemäß veränderten Pyruvatcarboxylase von Sequenz Nr.3. Ganz besonders bevorzugt ist die Pyruvatcarboxylase nach Sequenz Nr. 3.

Durch Expression des Gens nach Sequenz Nr. 2 wird eine erfindungsgemäße Pyruvatcarboxylase mit einer mindestens 90%igen Sequenzidentität zu der Pyruvatcarboxylase nach Sequenz Nr. 4 erhalten, bei welcher ausgehend von der Pyruvatcarboxylase des Stammes ATCC 13032 *lysC*^{T311I} das Threonin in Position 343 durch Alanin ersetzt ist und zusätzlich das Isoleucin an Position 1012 durch Serin ersetzt ist (*pyc*^{T343A;I1012S}). In Position 343 der Pyruvatcarboxylase befindet sich daher erfindungsgemäß Alanin und in Position 1012 Serin. Von der erfindungsgemäßen Pyruvatcarboxylase sind Pyruvatcarboxylasen einer 90%igen bis 100%igen Identität zu der Sequenz Nr. 4 umfasst. Vorzugsweise ist die Identität 95%, 96% oder 97%, besonders bevorzugt 98% oder 99% der erfindungsgemäß veränderten Pyruvatcarboxylase von Sequenz Nr. 4. Ganz besonders bevorzugt ist die Pyruvatcarboxylase nach Sequenz Nr. 4.

Der Ausdruck Identität wie hierin verwendet, kann durch die Gleichung I (%) = [1-V/X] × 100, definiert werden, worin I Identität bedeutet, X die Gesamtzahl an Aminosäuren der Vergleichssequenz ist und V die Anzahl an unterschiedliche Aminosäuren der zu betrachtenden Sequenz bezogen auf die Vergleichssequenz ist. Folgende Sequenzen sind im Sequenzprotokoll gelistet:
- Seq. Nr. 1:: Erfindungsgemäß veränderte DNA-Sequenz der Plasmid-basierten Variante, codierend für die erfindungsgemäß veränderte Pyruvatcarboxylase *pyc*^{T343A}
- Seq. Nr. 2:: Erfindungsgemäß veränderte DNA-Sequenz der Plasmid-basierten Variante, codierend für die erfindungsgemäß veränderte Pyruvatcarboxylase *pyc*^{T343A;I1012S}.
- Seq. Nr. 3:: Aminosäuresequenz der erfindungsgemäß veränderten Pyruvatcarboxylase Pyc-T343A.
- Seq. Nr. 4:: Aminosäuresequenz der erfindungsgemäß veränderten Pyruvatcarboxylase Pyc-T343A;I1012S.
- Seq. Nr. 5:: DNA-Sequenz des Referenzstammes ATCC 13032 *lysC*^{T311I} für die Wildform der Pyruvatcarboxylase.
- Seq. Nr. 6:: Aminosäuresequenz der Pyruvatcarboxylase des Referenzstammes ATCC 13032 *lysC*^{T311I} für die Wildform der Pyruvatcarboxylase.
- Seq. Nr. 7:: DNA-Sequenz der erfindungsgemäßen chromosomalen Variante DNA-*pyc-*A1027G-T1035G codierend für Pyc-T343A.
- Seq. Nr. 8:: DNA-Sequenz der erfindungsgemäßen chromosomalen Variante DNA-*pyc-*A1027G-T1035G-T3035G-C3039G codierend für Pyc-T343A;I1012S.

In einer vorteilhaften Weiterbildung der Erfindung kann die Expression der erfindungsgemäßen Gene verstärkt werden. Dazu können beispielhaft aber nicht beschränkend stärkere Promotoren verwendet werden, die Anzahl der Genkopien kann erhöht werden oder es kann die Ribosomenbindestelle verändert werden, um die Translation der Boten-RNA zu steigern. Die für die Durchführung dieser Methoden zu verwendenden Verfahren sind dem Fachmann bekannt.

Weiterhin ist ein Mikroorganismus Gegenstand der Erfindung, welcher ein erfindungsgemäßes Gen oder einen erfindungsgemäßen Vektor enthält. Dieser Mikroorganismus ist vorzugsweise ein coryneformes Bakterium. Als coryneforme Bakterien können beispielsweise *Corynebacterium glutamicum, Corynebacterium acetoglutamicum, Corynebacterium acetoacidophilum, corynebacterium melassecola, Corynbacterium thermoaminogenes, Corynebacterium efficiens, Brevibacterium flavum oder Brevibacterium lactofermentum* genannt werden.
Erfindungsgemäß besonders bevorzugte Zellen sind diejenigen der Gattungen *Corynebacterium*, *Brevibacterium*, *Escherichia*, *Bacillus*, *Lactobacillus*, *Lactococcus*, *Zymomonas*, *Methylobacterium*, *Ralstonia*, *Clostridium*, *Candida*, *Pichia*, *Kluyveromyces*, *Saccharomyces* und *Yarrowia*, wobei *Corynebacterium glutamicum*, *Corynebacterium efficiens*, *Brevibacterium flavum*, *Brevibacterium lactofermentum*, *Escherichia coli*, *Saccharomyces cerevisiae*, *Kluyveromyces lactis*, *Candida blankii*, *Candida rugosa*, *Zymomonas mobilis*, *Yarrowia lipolytica*, *Methylobacterium extorquens*, *Ralstonia eutropha* und *Pichia pastoris* besonders bevorzugt sind. Erfindungsgemäß am meisten bevorzugte Zellen sind solche der Gattung *Corynebacterium* und *Escherichia*, wobei *Corynebacterium glutamicum* und *Escherichia coli* ganz besonders bevorzugte Bakterienstämme sind.

Insbesondere in dem Fall, in dem das Produkt L-Lysin ist, können sich die gentechnisch veränderten Zellen insbesondere von Zellen ausgewählt aus der Gruppe bestehend aus *Corynebacterium glutamicum* ATCC13032, *Corynebacterium acetoglutamicum* ATCC15806, *Corynebacterium acetoacidophilum* ATCC13870, *Corynebacterium melassecola* ATCC17965, *Corynebacterium thermoaminogenes* FERM BP-1539, *Brevibacterium flavum* ATCC14067, *Brevibacterium lactofermentum* ATCC13869 und *Brevibacterium divaricatum* ATCC14020, und daraus hergestellte L-Aminosäuren produzierende Mutanten bzw. Stämme wie beispielsweise die L-Lysin produzierenden Stämme *Corynebacterium glutamicum* FERM-P 1709, *Brevibacterium flavum* FERM-P 1708, *Brevibacterium lactofermentum* FERM-P 1712, *Corynebacterium glutamicum* FERM-P 6463, *Corynebacterium glutamicum* FERM-P 6464 und *Corynebacterium glutamicum* DSM 5715 oder wie beispielsweise der L-Methionin produzierende Stamm *Corynebacterium glutamicum* ATCC21608 ableiten. Als Beispiele geeigneter *Escherichia* coli-Stämme seien *Escherichia coli* AJ11442 (siehe JP 56-18596 und US 4,346,170), *Escherichia coli*-Stamm VL611 und *Escherichia coli*-Stamm WC196 (siehe WO-A-96/17930) genannt.

Erfindungsgemäß wird ein Verfahren zur Herstellung von Produkten, deren Biosynthese Oxalacetat als Vorstufe beinhaltet, zur Verfügung gestellt.

Dazu wird ein Mikroorganismus, enthaltend ein für die Pyruvatcarboxylase codierendes Gen mit der genetischen Veränderung, kodierend für den Austausch T343A oder zwei Austausche T343A und I1012S im Protein, für die Produktion von aus Oxalacetat abgeleiteten Stoffwechselprodukten verwendet.

Hierzu wird ein Gen mit einer mindestens 70%igen Identität zu der Sequenz Nr. 1, bei der in den Positionen 1027-1029 das für Threonin kodierende Triplett gegen ein Triplett kodierend für Alanin ausgetauscht ist, oder ein Gen mit einer mindestens 70%igen Identität zu der Sequenz Nr. 2, bei der in den Positionen 1027-1029 das für Threonin kodierende Triplett gegen ein Triplett kodierend für Alanin ausgetauscht ist und zusätzlich in Position 3034-3036 das für Isoleucin kodierende Triplett gegen ein Triplett kodierend für Serin ausgetauscht ist, in den Mikroorganismus eingeführt.

Von dem erfindungsgemäßen Verfahren ist die Verwendung eines Gens mit einer mindestens 70%igen bis 100%igen Identität zu dem Gen nach Sequenz Nr. 1 oder 2 umfasst.

Vorzugsweise wird ein Mikroorganismus mit einem Gen einer Identität von mindestens 80% bis 90% zu Sequenz Nr. 1 oder Seq. Nr. 2 verwendet. Besonders bevorzugt wird ein Gen mit einer Identität von 91%, 92%, 93%, 94%, 95%, 96%, 97%, 98% oder 99% zu Sequenz Nr. 1 oder Sequenz Nr. 2 für das Herstellungsverfahren verwendet. Ganz besonders bevorzugt ist das Gen nach Sequenz Nr. 1 oder Sequenz Nr. 2.

Das erfindungsgemäß eingesetzte für die Pyruvatcarboxylase kodierende Gen kann dabei chromosomal oder in einem Vektor, vorzugsweise einem Plasmid, verwendet werden.

Das Gen wird exprimiert und die erfindungsgemäße Pyruvatcarboxylase mit einer Identität von mindestens 90% zu der Pyruvatcarboxylase nach Sequenz Nr. 3, bei der in Position 343 Threonin durch Alanin ersetzt ist, bewirkt die erhöhte Produktion von Oxalacetat abgeleiteten Stoffwechselprodukten.

Auch die Expression der erfindungsgemäßen Pyruvatcarboxylase mit einer Identität von mindestens 90% zu der Pyruvatcarboxylase nach Sequenz Nr. 4, bei der in Position 343 Threonin durch Alanin und in Position 1012 Isoleucin durch Serin ersetzt ist, bewirkt die erhöhte Produktion von Oxalacetat abgeleiteten Stoffwechselprodukten.

Von dem erfindungsgemäßen Verfahren ist die Verwendung einer Pyruvatcarboxylase mit einer 90%igen bis 100%igen Identität zu der Pyruvatcarboxylase nach Sequenz Nr. 3 und Sequenz Nr. 4 umfasst.

Vorzugsweise ist die Identität der erfindungsgemäß verwendeten Pyruvatcarboxylase 95%, 96% oder 97%, besonders bevorzugt 98% oder 99% zu Sequenz Nr. 3 oder 4. Besonders bevorzugt ist die Verwendung der Pyruvatcarboxylase nach Sequenz Nr. 3. Ganz besonders bevorzugt ist die Verwendung der Pyruvatcarboxylase nach Sequenz Nr. 4.

In einer bevorzugten Ausführungsform wird das erfindungsgemäße Gen verstärkt exprimiert.

Die so erhaltenen Mikroorganismen werden fermentiert.

Als Produktionsorganismus kann vorzugsweise ein Organismus aus der Gruppe bestehend aus den Gattungen *Corynebacterium*, *Brevibacterium*, *Escherichia*, *Bacillus*, *Lactobacillus*, *Lactococcus*, *Zymomonas*, *Methylobacterium*, *Ralstonia*, *Clostridium*, *Candida*, *Pichia*, *Kluyveromyces*, *Saccharomyces* und *Yarrowia*, wobei *Corynebacterium glutamicum*, *Corynebacterium efficiens*, *Brevibacterium flavum*, *Brevibacterium lactofermentum*, *Escherichia coli*, *Saccharomyces cerevisiae*, *Kluyveromyces lactis*, *Candida blankii*, *Candida rugosa, Zymomonas mobilis, Yarrowia lipolytica, Methylobacterium extorquens*, *Ralstonia eutropha* und *Pichia pastoris* besonders bevorzugt sind, eingesetzt werden. Erfindungsgemäß am meisten bevorzugte Zellen sind solche der Gattung *Corynebacterium* und *Escherichia*, wobei *Corynebacterium glutamicum* und *Escherichia coli* ganz besonders bevorzugte Bakterienstämme sind.

Insbesondere in dem Fall, in dem der Metabolit L-Lysin ist, können die gentechnisch veränderten Zellen bzw. Mikroorganismen insbesondere von Zellen ausgewählt aus der Gruppe bestehend aus *Corynebacterium glutamicum* ATCC13032, *Corynebacterium acetoglutamicum* ATCC15806, *Corynebacterium acetoacidophilum* ATCC13870, *Corynebacterium melassecola* ATCC17965, *Corynebacterium thermoaminogenes* FERM BP-1539, Brevibacterium *flavum* ATCC14067, *Brevibacterium lactofermentum* ATCC13869 und *Brevibacterium divaricatum* ATCC14020, und daraus hergestellte L-Aminosäuren produzierende Mutanten bzw. Stämme wie beispielsweise die L-Lysin produzierenden Stämme *Corynebacterium glutamicum* FERM-P 1709, *Brevibacterium flavum* FERM-P 1708, *Brevibacterium lactofermentum* FERM-P 1712, *Corynebacterium glutamicum* FERM-P 6463, *Corynebacterium glutamicum* FERM-P 6464 und *Corynebacterium glutamicum* DSM 5715 oder wie beispielsweise der L-Methionin produzierende Stamm *Corynebacterium glutamicum* ATCC21608 ableiten. Als Beispiele geeigneter *Escherichia coli*-Stämme seien *Escherichia coli* AJ11442 (siehe JP 56-18596 und US 4,346,170), *Escherichia coli*-Stamm VL611 und *Escherichia coli-*Stamm WC196 (siehe WO-A-96/17930) eingesetzt werden.

Mit dem erfindungsgemäßen Verfahren können insbesondere Aminosäuren der Aspartat-Familie gesteigert werden, also L-Lysin, L-Aspartat, L-Asparagin, L-Threonin, L-Isoleucin und L-Methionin hergestellt werden. Weiterhin kann die Produktion von Aminosäuren der Glutamat-Familie, wie L-Glutamat, L-Glutamin, L-Arginin oder L-Prolin, von Intermediaten des Citrat-Zyklus, wie z.B. Succinat, Fumarat, Malat, Citrat, Isocitrat oder 2-Oxoglutarat, von Diaminen z.B. Diaminopentan oder Diaminobutan oder auch anderen Produkten wie Itaconat, Ectoin, gamma-Aminobutyrat, Butanol, 1-Propanol, L-Citrullin, L-Ornithin, D-Arginin oder 4-Hydroxyprolin gesteigert werden.

Das durch Fermentation hergestellte und in den Kulturüberstand sekretierte Produkt wird dann angereichert und isoliert.

Im Folgenden werden experimentelle Ergebnisse anhand von Figuren dargestellt, die die Erfindung exemplarisch wiedergeben.

Es zeigt:
- Fig. 1:: Wachstum von *C. glutamicum* ATCC 13032 *lysC*^{T311I} mit nativer Pyc, chromosomal codierter Pyc^{T343A} und chromosomal codierter Pyc^{T343A;I1012S}.
- Fig. 2:: L-Lysin-Produktion der Stämme *C. glutamicum* ATCC 13032 *lys*C^{T311I}, *C. glutamicum* ATCC 13032 *lysC*^{T311I} mit chromosomal codierter Pyc^{T343A}, sowie *C. glutamicum* ATCC 13032 *lys*C^{T311I} mit chromosomal codierter Pyc^{T343A;I1012S}.
- Fig. 3:: Wachstum von *C. glutamicum* ATCC 13032 *lysC*^{T311I} Δ*pyc* mit dem Plasmid pAN6-*pyc*^{T343A;I1012S}
- Fig. 4:: L-Lysin-Produktion des Stammes *C. glutamicum* ATCC 13032 *lys*C^{T311I} Δ*pyc* mit dem Plasmid pAN6-*pyc*^{T343A;I1012S}.

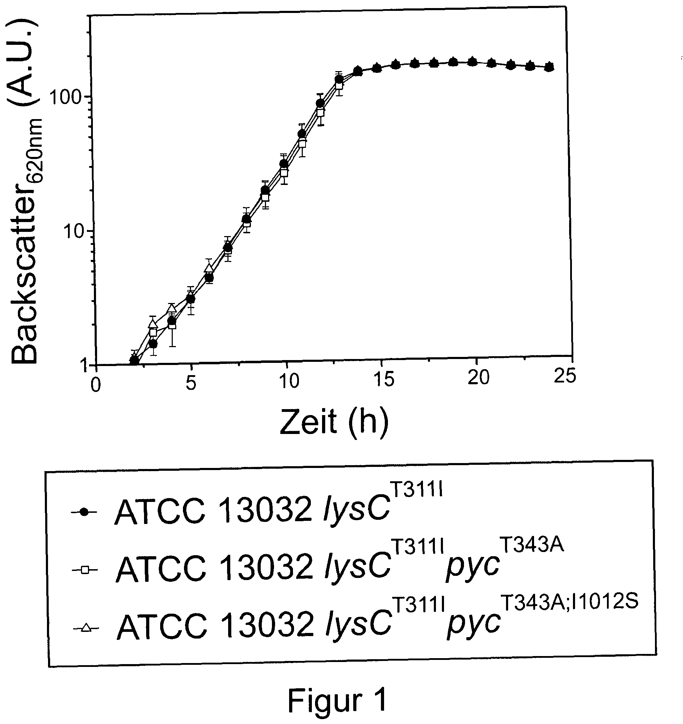

In Figur 1 wird das Wachstum des Stammes C. *glutamicum* ATCC 13032 *lys*C^{T311I} mit den chromosomal codierten Pyc-Varianten Pyc^{T343A} und Pyc^{T343A;I1012S} dargestellt. In Figur 1 zeigt die Abszisse die Zeit in Stunden (h) und die Ordinate den Wert für Backscatter bei 620 nm (A.U.) als Maß der Zelldichte. Als Kontrolle diente der Stamm C. *glutamicum* ATCC 13032 *lys*C^{T311I} mit nativer Pyc, also mit Threonin an Position 343 und Isoleucin an Position 1012. Alle Stämme wurden in CGXII Minimalmedium mit 4% (wt/vol) Glucose in einem BioLector® System bei 30 °C und 1200 rpm für 24 h kultiviert.

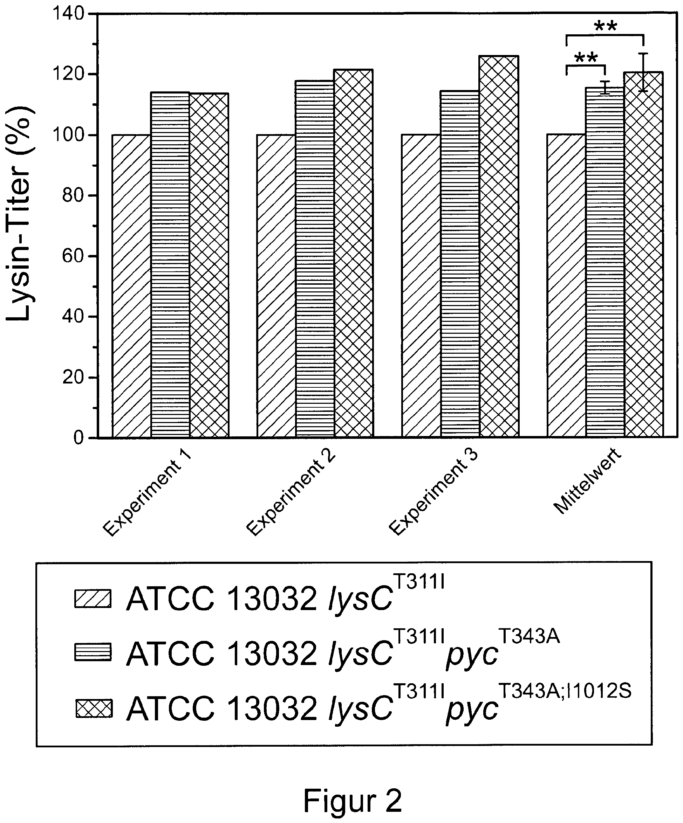

Figur 2 zeigt die L-Lysin-Produktion der Stämme C. *glutamicum* ATCC 13032 *lysC*^{T311I} mit der chromosomal codierten Pyc-Variante Pyc^{T343A}und mit der chromosomal codierten Pyc-Variante Pyc^{T343A;I1012S}. Darin bezeichnet die Abszisse drei unabhängige Replikate sowie den Mittelwert aus den drei Experimenten und die Ordinate die prozentuale Lysin-Konzentration, wobei die Lysin-Konzentration des Kontrollstammes ATCC 13032 *lys*C^{T311I} (schwarze Balken) in den drei unabhängigen Replikaten jeweils als 100% gesetzt wurde. Die L-Lysin Konzentrationen für den Stamm ATCC 13032 *lys*C^{T311I} *pyc*^{T343A} sind als schräg schraffierte Balken dargestellt. Die L-Lysin Konzentrationen für den Stamm ATCC 13032 *lysC*^{T311I} *pyc*^{T343A;I1012S} sind als waagerecht schraffierte Balken dargestellt. Nach Kultivierung der Zellen in einem BioLector® System für 24 h (s. Fig.1) wurden die Zellen geerntet und die L-Lysin Konzentration im Überstand der jeweiligen Kulturen via reversed-phase HPLC mit ortho-Phthaldialdehyd-Derivatisierung bestimmt. In jedem der drei Replikate zeigte sowohl der Stamm mit der mutierten Pyc-Variante Pyc^{T343A}als auch der Stamm mit der mutierten Pyc-Variante *pyc*^{T343A;I1012S} einen höheren Lysin-Titer als der Vergleichsstamm mit nativer Pyc. Es ist eine Steigerung der finalen L-Lysin-Konzentration um durchschnittlich 15-19% gegenüber C. *glutamicum* ATCC 13032 *lys*C^{T311I} zu beobachten. Die gemessenen Lysin-Konzentrationen der einzelnen Mutanten wurden mittels t-Test verglichen. Dabei stehen die in der Abbildung gezeigten Sternchen für p ≤ 0,01.

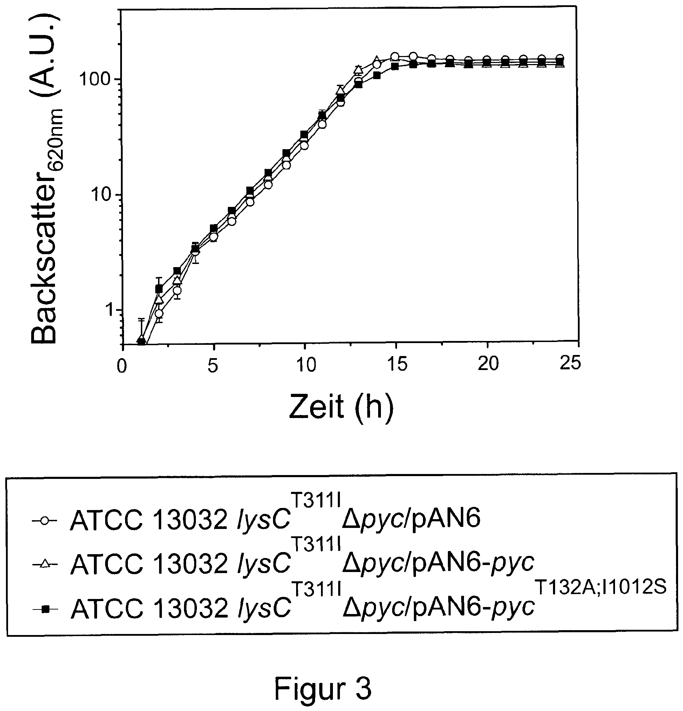

In Figur 3 ist das Wachstum des Stammes C. *glutamicum* ATCC 13032 *lys*C^{T311I} Δ*pyc* mit dem Plasmid pAN6-*pyc*^{T343A;I1012S} dargestellt. In der Figur zeigt wiederum die Abszisse die Zeit in Stunden (h) und die Ordinate den Wert für Backscatter bei 620 nm (A.U.). Zur Kontrolle wurden die Stämme C. *glutamicum* ATCC 13032 *lysC*^{T311I} Δ*pyc*, welche entweder das Leerplasmid pAN6 oder das Plasmid pAN6-pyc mit nativer *pyc* tragen, mitgeführt. Alle Stämme wurden in CGXII Minimalmedium mit 4% (wt/vol) Glucose und 25 mg L⁻¹ Kanamycin in einem BioLector® System bei 30 °C und 1200 rpm für 24 h kultiviert. Zu Beginn der Kultivierung wurde die Expression von *pyc* mit 1 mM IPTG (Isopropyl-ß-D-thiogalactosid) induziert.

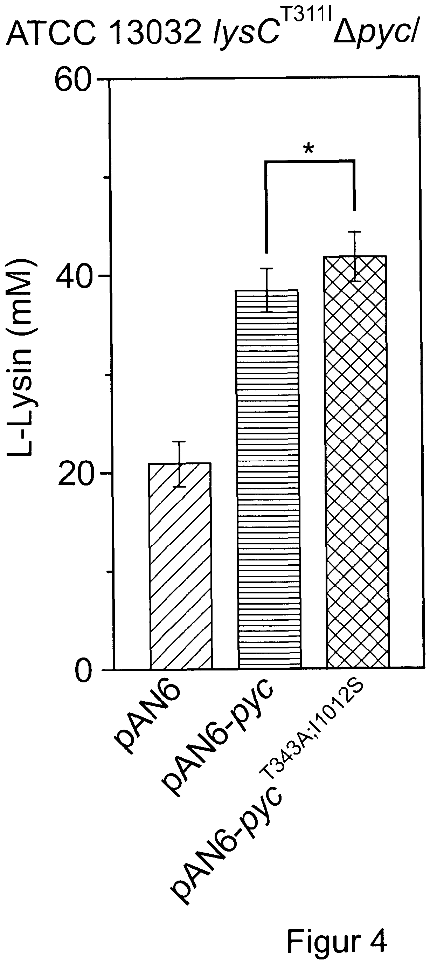

Figur 4 zeigt die L-Lysin-Produktion des Stammes C. *glutamicum* ATCC 13032 *lys*C^{T311I} Δ*pyc* mit dem Plasmid pAN6- *pyc*^{T343A;I1012S} Darin bezeichnet die Abszisse den Stamm C. *glutamicum* ATCC 13032 *lys*C^{T311I} Δ*pyc* enthaltend den Leervektor pAN6 (schraffierter Balken), den Vektor pAN6-pyc mit nativer Pyruvatcarboxylase (weißer Balken) oder den Vektor pAN6-*pyc*^{T343A;I1012S} (schwarzer Balken) im Vergleich. Nach Kultivierung der Zellen in einem BioLector® System für 24 h (s. Fig. 3), wurden die Zellen geerntet und die L-Lysin Konzentration im Überstand der jeweiligen Kulturen via reversed-phase HPLC mit ortho-Phthaldialdehyd-Derivatisierung bestimmt. Als Vergleich wurde die Lysin-Produktion im Stamm C. *glutamicum* ATCC 13032 *lys*C^{T311I} Δ*pyc*/pAN6 (ohne Pyc) und ATCC 13032 *lys*C^{T31I}1/pAN6-pyc (mit nativer Pyc) gemessen. Die vorliegenden Daten stellen Mittelwerte und Standardabweichungen aus mindestens sechs biologischen Replikaten dar. Die gemessenen Lysin-Konzentrationen der einzelnen Mutanten wurden mittels t-Test verglichen. Dabei steht das in der Abbildung gezeigte Sternchen für p ≤0.05. Es ist eine deutliche Erhöhung der finalen L-Lysin-Konzentration um 9% in dem Stamm mit der Pyc^{T343A;I1012S}-Variante im Vergleich zu dem Stamm mit nativer Pyc zu erkennen.

Im Rahmen der Erfindung konnte eine Mutation im *pyc*-Gen identifiziert werden, die zu einer erhöhten L-Lysin-Produktion führt. Es wurde zunächst mittels error prone-PCR eine Plasmid-basierte Pyc-Mutanten-Bibliothek erstellt, die anschließend im Stamm ATCC1303 *lys*C^{T311I} Δ *pyc* mit Hilfe eines genetisch kodierten Lysin-Sensors (pSenLys) und Fluoreszenzaktivierter Zellsortierung (FACS) zunächst auf erhöhte Fluoreszenz gescreent wurde. Die isolierten Zellen wurden anschließend vermehrt und auf erhöhte Lysinbildung getestet. Diese Methoden sind dem Fachmann bekannt (vgl. Binder et al., Genome Biol. 2012, 13:R40). Die dabei isolierten Gen- und Enzymvarianten wurden genetisch charakterisiert, was schließlich zur Identifizierung der erfindungsgemäßen Pyc-Variante führte, welche die Produktion von L-Lysin, sowie anderen, von Oxalacetat abgeleiteten Metaboliten, steigert. Die gefundenen Mutationen lauten T343A und T343A;I1012S.

### 1. Chromosomal kodierte Pvc-Variante Pvc-T343A:

### Messung der L-Lysin-Produktion von C. glutamicum ATCC 13032 lysC^{T311I} mit der chromosomal kodierten Pyc-Variante Pyc-T343A:

Der Austausch des Threonin-Codons 343 im chromosomalen *pyc*-Gen durch ein Alanin-Codon erfolgte mittels zweifacher homologer Rekombination unter Verwendung des Vektors pK19mobsacB nach dem Fachmann bekannten Verfahren (Schäfer et al. 1994 Gene 145:69-73; Hochheim et al. 2017 Biotechnol Lett 39:283-288). Verglichen wurde die Produktion mit dem Stamm *C. glutamicum* ATCC 13032 *lysC*^{T311I} mit nativem chromosomalem *pyc*-Gen. Die Stämme wurden in 800 µl CGXII-Minimalmedium mit 4% (wt/vol) Glucose für 24h in einem Biolector-System bei 30°C und 1200 rpm kultiviert (Fig.1). Die Start-OD bei 600 nm betrug 0,5. Nach 24 h wurden die Zellen der einzelnen Kulturen sedimentiert und die L-Lysin-Konzentration im Überstand gemessen. Die Messung erfolgte über reversed phase HPLC mit einer Vorsäulenderivatisierung der Aminosäuren über ortho-Phthaldialdehyd. Als mobile Phase wurde ein Gradient von 80% Lösung A (100 mM Natrium-Acetat (pH 7,2)) und 20% Lösung B (100% (vol/vol) Methanol) zu 20% Lösung A und 80% Lösung B verwendet. Das Beispiel zeigt, dass die untersuchte Pyc-Variante einen positiven Effekt auf die Lysin-Produktion hat und diese bis zu 15% gesteigert werden kann, wenn die Pyc-Variante Pyc-T343A anstelle des wildtypischen Pyc-Proteins chromosomal codiert ist.

### 2. Chromosomal kodierte Pvc-Variante Pyc-T343A;I1012S:

### Messung der L-Lysin-Produktion von C. glutamicum ATCC 13032 lysC^{T311I} mit der chromosomal kodierten Pyc-Variante Pyc-T343A;I1012S:

Der Austausch des Threonin-Codons 343 durch ein Alanin-Codon und des Isoleucin-Codons 1012 durch ein Serin-Codon im chromosomalen pyc-Gen erfolgte mittels zweifacher homologer Rekombination unter Verwendung des Vektors pK19mobsacB nach dem Fachmann bekannten Verfahren (Schäfer et al. 1994 Gene 145:69-73; Hochheim et al. 2017 Biotechnol Lett 39:283-288). Verglichen wurde die Produktion mit dem Stamm C. *glutamicum* ATCC 13032 *lysC*^{T311I} mit nativem chromosomalem *pyc*-Gen. Die Stämme wurden in 800 µl CGXII-Minimalmedium mit 4% (wt/vol) Glucose für 24h in einem Biolector-System bei 30°C und 1200 rpm kultiviert (Fig.1). Die Start-OD bei 600 nm betrug 0,5. Nach 24 h wurden die Zellen der einzelnen Kulturen sedimentiert und die L-Lysin-Konzentration im Überstand gemessen. Die Messung erfolgte über reversed phase HPLC mit einer Vorsäulenderivatisierung der Aminosäuren über ortho-Phthaldialdehyd. Als mobile Phase wurde ein Gradient von 80% Lösung A (100 mM Natrium-Acetat (pH 7,2)) und 20% Lösung B (100% (vol/vol) Methanol) zu 20% Lösung A und 80% Lösung B verwendet. Das Beispiel zeigt, dass die untersuchte Pyc-Variante einen positiven Effekt auf die Lysin-Produktion hat und diese bis zu 19% gesteigert werden kann, wenn die Pyc-Variante Pyc-T343A;I1012S anstelle des wildtypischen Pyc-Proteins chromosomal codiert ist.

### 3. Plasmid-kodierte Pvc-Variante Pyc-T343A;I1012S:

### Messung der L-Lysin-Produktion von C. glutamicum ATCC 13032 lysC^{T311I} Δpyc/pAN6-pyc^{T343A;I1012S}:

Verglichen wurde die Produktion mit dem Stamm *C. glutamicum* ATCC 13032 *lys*C^{T311I}Δ*pyc*/pAN6-*pyc* codierend die native Pyc und der Leerplasmidkontrolle C. *glutamicum* ATCC 13032 *lys*C^{T311I}Δ*pyc*/pAN6. Die Stämme wurden in 800 µl CGXII-Minimalmedium mit 4% (wt/vol) Glucose und 25 mg/l Kanamycin für 24 h in einem Biolector-System bei 30°C und 1200 rpm kultiviert (Fig.3). Die Start-OD bei 600 nm betrug 0,5. Nach 24 h wurden die Zellen der einzelnen Kulturen sedimentiert und die L-Lysin-Konzentration im Überstand gemessen. Die Messung erfolgte über reversed phase HPLC mit einer Vorsäulenderivatisierung der Aminosäuren über ortho-Phthaldialdehyd. Als mobile Phase wurde ein Gradient von 80% Lösung A (100 mM Natrium-Acetat (pH 7,2)) und 20% Lösung B (100% (vol/vol) Methanol) zu 20% Lösung A und 80% Lösung B verwendet. Das Beispiel zeigt, dass die Lysin-Produktion um bis zu 9% gesteigert werden kann, wenn die Pyc-Variante *pyc*^{T343A;I1012S} anstelle der wildtypischen Pyc auf dem Plasmid pAN6 codiert ist. (Fig.4)

## Patentansprüche

1. DNA-Sequenz kodierend für eine Pyruvatcarboxylase mit einer mindestens 70%igen Identität zu der Sequenz Nr. 1, wobei das Triplett in Position 1027-1029 für Alanin kodiert.

2. DNA-Sequenz nach Anspruch 1,
**dadurch gekennzeichnet,**
**dass** die DNA eine Identität von mindestens 80% zu der Sequenz Nr. 1 besitzt.

3. DNA-Sequenz nach einem der Ansprüche 1 oder 2,
**dadurch gekennzeichnet,**
**dass** sie eine DNA nach Sequenz Nr. 1 ist.

4. DNA Sequenz nach einem der Ansprüche 1 bis 3,
**dadurch gekennzeichnet,**
**dass** sich zusätzlich in Position 3034-3036 ein Triplett befindet, welches für Serin kodiert.

5. DNA-Sequenz nach Anspruch 4,
**dadurch gekennzeichnet,**
**dass** die DNA eine Identität von mindestens 80% zu der Sequenz Nr. 2 besitzt.

6. DNA-Sequenz nach Anspruch 4 oder 5,
**dadurch gekennzeichnet,**
**dass** die eine DNA nach Sequenz Nr. 2 ist.

7. Pyruvatcarboxylase mit einer mindestens 90%igen Identität zu der Pyruvatcarboxylase nach Sequenz Nr. 3, bei der sich in Position 343 Alanin befindet.

8. Pyruvatcarboxylase nach Anspruch 7,
**dadurch gekennzeichnet,**
**dass** die Pyruvatcarboxylase eine Identität von mindestens 95% zu der Pyruvatcarboxylase nach Sequenz Nr. 3 besitzt.

9. Pyruvatcarboxylase nach Anspruch 7 oder 8,
**dadurch gekennzeichnet,**
**dass** sie eine Pyruvatcarboxylase nach Sequenz Nr. 3 ist.

10. Pyruvatcarboxylase nach einem der Ansprüche 6 bis 8,
**dadurch gekennzeichnet,**
**dass** sie zusätzlich in Position 1012 Serin hat.

11. Pyruvatcarboxylase nach Anspruch 10,
**dadurch gekennzeichnet,**
**dass** die Pyruvatcarboxylase eine Identität von mindestens 95% zu der Pyruvatcarboxylase nach Sequenz Nr. 4 besitzt.

12. Pyruvatcarboxylase nach Anspruch 10 oder 11,
**dadurch gekennzeichnet,**
**dass** sie eine Pyruvatcarboxylase nach Sequenz Nr. 4 ist.

13. Vektor,
**dadurch gekennzeichnet,**
**dass** er eine DNA-Sequenz nach einem der Ansprüche 1 bis 6 enthält.

14. Vektor nach Anspruch 13,
**dadurch gekennzeichnet,**
**dass** er ein Plasmid ist.

15. Mikroorganismus,
**dadurch gekennzeichnet,**
**dass** er eine DNA nach einem der Ansprüche 1 bis 6 enthält.

16. Mikroorganismus nach Anspruch 9,
**dadurch gekennzeichnet,**
**dass** er einen Vektor nach einem der Ansprüche 13 oder 14 enthält.

17. Mikroorganismus nach Anspruch 15 oder 16,
**dadurch gekennzeichnet,**
**dass** er ein Mikroorganismus aus der Gruppe bestehend aus den Gattungen *Corynebacterium*, *Brevibacterium*, *Bacillus*, *Lactobacillus*, *Lactococcus*, *Candida*, *Pichia*, *Kluyveromyces*, *Saccharomyces*, *Escherichia*, *Zymomonas*, *Yarrowia*, *Methylobacterium*, *Raltonia*, *Vibrio* und *Clostridium* ist.

18. Verfahren zur Herstellung von Produkten, deren Biosynthese Oxalacetat als Vorstufe beinhaltet,
**dadurch gekennzeichnet,**
**dass** ein Mikroorganismus, enthaltend eine DNA nach den Ansprüchen 1 bis 6, der eine Pyruvatcarboxylase mit einer Sequenz nach einem der Ansprüche 7 bis 12 bildet, fermentiert wird, eine Anreicherung des Produktes im Medium oder in den Zellen durchgeführt wird.

19. Verfahren nach Anspruch 18,
**dadurch gekennzeichnet,**
**dass** das Produkt isoliert wird.

20. Verfahren nach Anspruch 18 oder 19,
**dadurch gekennzeichnet,**
**dass** als Mikroorganismus eine Spezies aus der Gruppe bestehend aus den Gattungen *Corynebacterium*, *Brevibacterium*, *Bacillus*, *Lactobacillus*, *Lactococcus*, *Candida*, *Pichia*, *Kluyveromyces*, *Saccharomyces*, *Escherichia*, *Zymomonas*, *Yarrowia*, *Methylobacterium*, *Raltonia*, *Vibrio* und *Clostridium* eingesetzt wird.

21. Verfahren nach einem der Ansprüche 18 bis 20,
**dadurch gekennzeichnet,**
**dass** eine Aminosäure der Aspartat-Familie hergestellt wird.

22. Verfahren nach Anspruch 21,
**dadurch gekennzeichnet,**
**dass** L-Lysin, L-Aspartat, L-Asparagin, L-Threonin, L-Isoleucin oder L-Methionin hergestellt wird.

23. Verfahren nach einem der Ansprüche 18 bis 20,
**dadurch gekennzeichnet,**
**dass** Stoffe aus der Glutamat-Familie der Aminosäuren wie L-Glutamat, Glutamin L-Arginin, L-Prolin, L-Citrullin oder L-Ornithin, Intermediate des Citrat-Zyklus, wie Malat, Fumarat, Succinat, Malat, Fumarat Citrat, Isocitrat oder 2-Oxoglutarat, Diamine wie Diaminopentan oder Diaminobutan sowie weitere aus Oxalacetat hergestellte Stoffwechselprodukte, wie Itaconat, Ectoin, gamma-Aminobutyrat, Butanol, 1-Propanol, L-Citrullin, L-Ornithin, D-Arginin oder 4-Hydroxyprolin hergestellt werden.

24. Verfahren nach einem der Ansprüche 18 bis 23,
**dadurch gekennzeichnet,**
**dass** die Gene nach den Ansprüchen 1 bis 6 verstärkt exprimiert werden.

25. Chromosom eines Mikroorganismus einer Spezies aus der Gruppe bestehend aus den Gattungen *Corynebacterium*, *Brevibacterium*, *Bacillus*, *Lactobacillus*, *Lactococcus*, *Candida*, *Pichia*, *Kluyveromyces*, *Saccharomyces*, *Escherichia*, *Zymomonas*, *Yarrowia*, *Methylobacterium*, *Ralstonia*, *Vibrio* und *Clostridium,* enthaltend eine DNA-Sequenz nach einem der Ansprüche 1 bis 6.

## Claims

1. A DNA sequence encoding a pyruvate carboxylase with at least 70% identity to sequence No. 1, wherein the triplett, in positions 1027-1029, encodes alanine.

2. A DNA sequence according to claim 1,
**characterized in**
**that** the DNA has an identity of at least 80% to sequence No. 1.

3. A DNA sequence according to any one of claims 1 or 2,
**characterized in**
**that** it is a DNA according to sequence No. 1.

4. A DNA sequence according to any one of claims 1 to 3,
**characterized in**
**that** a triplet is also located in positions 3034-3036, which triplet encodes a serine.

5. A DNA sequence according to claim 4,
**characterized in**
**that** the DNA has an identity of at least 80% to sequence No. 2.

6. A DNA sequence according to claim 4 or 5,
**characterized in**
**that** it is a DNA according to sequence No. 2.

7. A pyruvate carboxylase with at least 90% identity to the pyruvate carboxylase according to sequence No. 3, where alanine is located in position 343.

8. A pyruvate carboxylase according to claim 7,
**characterized in**
**that** the pyruvate carboxylase has an identity of at least 95% to the pyruvate carboxylase according to sequence No. 3.

9. A pyruvate carboxylase according to claim 7 or 8,
**characterized in**
**that** it is a pyruvate carboxylase according to sequence No. 3.

10. A pyruvate carboxylase according to any one of claims 6 to 8,
**characterized in**
**that** it also has serine in position 1012.

11. A pyruvate carboxylase according to claim 10,
**characterized in**
**that** the pyruvate carboxylase has an identity of at least 95% to the pyruvate carboxylase according to sequence No. 4.

12. A pyruvate carboxylase according to claim 10 or 11,
**characterized in**
**that** it is a pyruvate carboxylase according to sequence No. 4.

13. A vector,
**characterized in**
**that** it contains a DNA sequence according to any one of claims 1 to 6.

14. A vector according to claim 13,
**characterized in**
**that** it is a plasmid.

15. A microorganism,
**characterized in**
**that** it contains a DNA according to any one of claims 1 to 6.

16. A microorganism according to claim 9,
**characterized in**
**that** it contains a vector according to any one of claims 13 or 14.

17. A microorganism according to claim 15 or 16,
**characterized in**
**that** it is a microorganism from the group consisting of the genera *Corynebacterium, Brevibacterium, Bacillus, Lactobacillus, Lactococcus, Candida, Pichia, Kluyveromyces, Saccharomyces, Escherichia, Zymomonas, Yarrowia, Methylobacterium*, *Ralstonia*, *Vibrio* and *Clostridium.*

18. A method for the production of products, the biosynthesis of which includes oxalacetate as precursor,
**characterized in**
**that** a microorganism, containing a DNA according to claims 1 to 6, which forms a pyruvate carboxylase having a sequence according to any one of claims 7 to 12, is fermented, an enrichment of the product in the medium or in the cells is carried out.

19. A method according to claim 18,
**characterized in**
**that** the product is isolated.

20. A method according to claim 18 or 19,
**characterized in**
**that**, as a microorganism, a species from the group consisting of the genera *Corynebacterium, Brevibacterium, Bacillus, Lactobacillus, Lactococcus, Candida, Pichia, Kluyveromyces, Saccharomyces, Escherichia, Zymomonas, Yarrowia*, *Methylobacterium*, *Ralstonia*, *Vibrio* and *Clostridium* is used.

21. A method according to any one of claims 18 to 20,
**characterized in**
**that** an amino acid of the aspartate family is produced.

22. A method according to claim 21,
**characterized in**
**that** L-lysine, L-aspartate, L-asparagine, L-threonine, L-isoleucine or L-methionine is produced.

23. A method according to any one of claims 18 to 20,
**characterized in**
**that** substances from the glutamate family of the amino acids such as L-glutamate, glutamine L-arginine, L-proline, L-citrulline or L-ornithine, intermediates of the citrate cycle, such as malate, fumarate, succinate, malate, fumarate citrate, isocitrate or 2-oxoglutarate, diamines such as diaminopentane or diaminobutane as well as additional metabolic products made from oxalacetate, such as itaconate, ectoine, gamma-aminobutyrate, butanol, I-propanol, L-citrulline, L-ornithine, D-arginine or 4-hydroxyproline are produced.

24. A method according to any one of claims 18 to 23,
**characterized in**
**that** the genes according to claims 1 to 6 are expressed in an amplified manner.

25. A chromosome of a microorganism of a species from the group consisting of the genera *Corynebacterium*, *Brevibacterium*, *Bacillus*, *Lactobacillus*, *Lactococcus*, *Candida*, *Pichia*, *Kluyveromyces*, *Saccharomyces*, *Escherichia*, *Zymomonas*, *Yarrowia*, *Methylobacterium*, *Ralstonia*, *Vibrio* and *Clostridium*, containing a DNA sequence according to any one of claims 1 to 6.

## Revendications

1. Séquence d'ADN codant pour une pyruvate carboxylase, dont au moins 70% est identique à la séquence N°1, le triplet en position 1027-1029 codant pour l'alanine.

2. Séquence d'ADN suivant la revendication 1,
**caractérisée**
**en ce que** l'ADN est identique à au moins 80% à la séquence N°1.

3. Séquence d'ADN suivant l'une des revendications 1 ou 2,
**caractérisée**
**en ce que** c'est un ADN suivant la séquence N°1.

4. Séquence d'ADN suivant l'une des revendications 1 à 3,
**caractérisée**
**en ce que** supplémentairement en position 3034-3036 se trouve un triplet qui code pour la sérine.

5. Séquence d'ADN suivant la revendication 4,
**caractérisée**
**en ce que** l'ADN est identique à au moins 80% à la séquence N°2.

6. Séquence d'ADN suivant la revendication 4 ou 5,
**caractérisée**
**en ce que** c'est un ADN suivant la séquence N°2.

7. Pyruvate carboxylase identique à au moins 90% à la pyruvate carboxylase suivant la séquence N°3, dans laquelle de l'alanine se trouve en la position 343.

8. Pyruvate carboxylase suivant la revendication 7,
**caractérisée**
**en ce que** la pyruvate carboxylase est identique à au moins 95% à la pyruvate carboxylase suivant la séquence N°3.

9. Pyruvate carboxylase suivant les revendications 7 et 8,
**caractérisée**
**en ce que** c'est une pyruvate carboxylase suivant la séquence N°3.

10. Pyruvate carboxylase suivant l'une des revendications 6 à 8
**caractérisée**
**en ce qu'**elle a supplémentairement de la sérine en position 1012.

11. Pyruvate carboxylase suivant la revendication 10,
**caractérisée**
**en ce que** la pyruvate carboxylase est identique à au moins 95% à la pyruvate carboxylase suivant la séquence N°4.

12. Pyruvate carboxylase suivant la revendication 10 ou 11,
**caractérisée**
**en ce que** c'est une pyruvate carboxylase suivant la séquence N°4.

13. Vecteur,
**caractérisé**
**en ce qu'**il contient une séquence d'ADN suivant l'une des revendications 1 à 6.

14. Vecteur suivant la revendication 13,
**caractérisé**
**en ce que** c'est un plasmide.

15. Micro-organisme,
**caractérisé**
**en ce qu'**il contient un ADN suivant l'une des revendications 1 à 6.

16. Micro-organisme suivant la revendication 9,
**caractérisé**
**en ce qu'**il contient un vecteur suivant l'une des revendications 13 ou 14.

17. Micro-organisme suivant l'une des revendications 15 ou 16,
**caractérisé**
**en ce que** c'est un micro-organisme du groupe constitué des genres *Corynebacterium*, *Brevibacterium*, *Bacillus*, *Lactobacillus*, *Lactococcus*, *Candida*, *Pichia*, *Kluyveramyces*, *Saccharomyces*, *Escherichia*, *Zymomonas*, *Yarrowia*, *Methylobacterium*, *Raltonia*, *Vibrio* et *Clostridium.*

18. Procédé de préparation de produits, dont la biosynthèse comprend de l'oxalacétate comme précurseur,
**caractérisé**
**en ce que** l'on fait fermenter un micro-organisme, contenant un ADN suivant les revendications 1 à 6, qui forme une pyruvate carboxylase ayant une séquence suivant l'une des revendications 7 à 12 et on effectue un enrichissement du produit dans le milieu ou dans les cellules.

19. Procédé suivant la revendication 18,
**caractérisé**
**en ce que** l'on isole le produit.

20. Procédé suivant la revendication 18 ou 19,
**caractérisé**
**en ce que** l'on utilise comme micro-organisme une espèce du groupe constitué des genres *Corynebacterium*, *Brevibacterium*, *Bacillus*, *Lactobacillus*, *Lactococcus*, *Candida*, *Pichia*, *Kluyveromyces*, *Saccharomyces*, *Escherichia*, *Zymomomas*, *Yarrowia*, *Methylobacterium*, *Ralstonia*, *Vibrio* et *Clostridium.*

21. Procédé suivant l'une des revendications 18 à 20,
**caractérisé**
**en ce que** l'on prépare un acide aminé de la famille de l'aspartate.

22. Procédé suivant la revendication 21,
**caractérisé**
**en ce que** l'on prépare la L-lysine, le L-aspartate, la L-asparagine, la L-thréonine, la L-isoleucine ou la L-méthionine.

23. Procédé suivant l'une des revendications 18 à 20,
**caractérisé**
**en ce que** l'on prépare des substances de la famille du glutamate des acides aminés, comme le L-glutamate, la glutamine L-arginine, la L-proline, la L-citruline ou la L-ornithine, intermédiaires du cycle citrate comme un maléate, un fumarate, un succinate, un maléate, un fumarate citrate, un isocitrate ou un 2-oxoglutarate, des diamines, comme le diaminopentane ou le diaminobutane, ainsi que d'autres produits du métabolisme fabriqués à partir de l'oxalacétate, comme l'itaconate, l'ectoïne, le gamma-aminobutyrate, le butanol, le 1-propanol, la L-citruline, la L-ornithine, la D-arginine ou la 4-hydroxyproline.

24. Procédé suivant l'une des revendications 18 à 23,
**caractérisé**
**en ce qu'**on exprime avec amplification les gènes suivant les revendications 1 à 6.

25. Chromosome d'un micro-organisme d'une espèce du groupe constitué des genres *Corynebacterium*, *Brevibacterium*, *Bacillus*, *Lactobacillus*, *Lactococcus*, *Candida*, *Pichia*, *Kluyveromyces*, *Saccharomyces*, *Escherichia*, *Zymomomas*, *Yarrowia*, *Methylobacterium*, *Ralstonia*, *Vibrio* et *Clostridium*, contenant une séquence d'ADN suivant l'une des revendications 1 à 6.
